(19) Europäisches Patentamt
European Patent Office
Office européen des brevets

(11) EP 4 455 298 A1

(12) EUROPEAN PATENT APPLICATION
published in accordance with Art. 153(4) EPC

(43) Date of publication:
**30.10.2024 Bulletin 2024/44**

(21) Application number: **22911362.6**

(22) Date of filing: **22.12.2022**

(51) International Patent Classification (IPC):
***C12Q 1/04*** *(2006.01)* ***C12N 5/0783*** *(2010.01)*
***C12N 5/10*** *(2006.01)* ***C12N 15/09*** *(2006.01)*
***G01N 33/50*** *(2006.01)*

(52) Cooperative Patent Classification (CPC):
**C12N 5/06; C12N 5/10; C12N 15/09; C12Q 1/04; G01N 33/50**

(86) International application number:
**PCT/JP2022/047423**

(87) International publication number:
**WO 2023/120660 (29.06.2023 Gazette 2023/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.12.2021 JP 2021209987**
**30.09.2022 JP 2022158190**

(71) Applicant: **Takeda Pharmaceutical Company Limited**
**Osaka-shi, Osaka 541-0045 (JP)**

(72) Inventors:
- **OGAKI, Soichiro**
**Fujisawa-shi, Kanagawa 251-0012 (JP)**
- **IIDA, Tomomine**
**Fujisawa-shi, Kanagawa 251-0012 (JP)**
- **SAITO, Shogo**
**Fujisawa-shi, Kanagawa 251-0012 (JP)**
- **ARAKI, Hideo**
**Fujisawa-shi, Kanagawa 251-0012 (JP)**

(74) Representative: **Jones, Nicholas Andrew**
**Withers & Rogers LLP**
**2 London Bridge**
**London SE1 9RA (GB)**

Remarks:
The complete document including Reference Table(s) and the Sequence Listing(s) can be downloaded from the EPO website

(54) **METHOD FOR PREDICTING GENE TRANSFER RATE**

(57) Disclosed is a method for predicting the gene transfer efficiency of animal cells, comprising (1) measuring the size of transgenic animal cells, and (2) predicting the gene transfer efficiency based on values measured in step (1).

Fig. 1

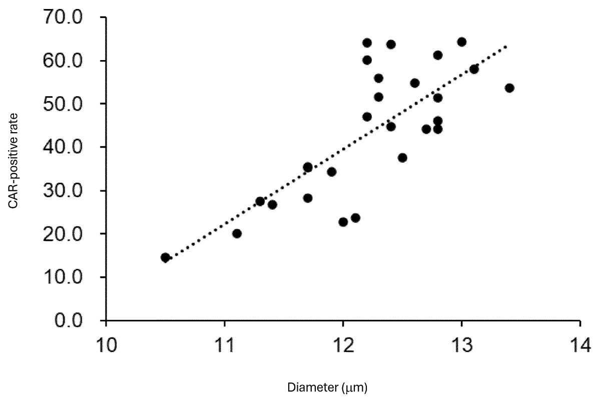

## Description

### Technical Field

The present invention relates to a method for predicting gene transfer efficiency.

### Background of Invention

In recent years, research and development of gene recombinant cellular medicines (transgenic/transfected cell preparations), such as chimeric antigen receptor (CAR)-T cells, has been actively conducted. For gene recombinant cellular medicines, such as CAR-T cells, the amount of the product is defined by multiplying the overall cell count by the percentage of transgene. Therefore, for CAR-T cell products, the CAR transfer rate is the most important index in the production process, shipping, and dosing. Conventionally, flow cytometry has been used to measure cell gene transfer efficiency; however, there is a problem that it takes a long time (6 to 7 hours) from measurement to analysis, and requires complicated steps (manual cell staining step). For example, NPL 1 has reported that CAR-T cells were measured by flow cytometry.

### Citation List

### Non-patent Literature

NPL 1: Cytometry Part B: Clinical Cytometry, Volume 100, Issue 2, pp. 218-224

### Summary of Invention

### Technical Problem

An object of the present invention is to provide a method for predicting gene transfer efficiency, the method being capable of measuring the gene transfer efficiency of cells more simply and efficiently than conventional methods.

### Solution to Problem

As a result of extensive studies to achieve the above object, the present inventors found that by measuring the cell size (e.g., diameter) and using it as an index, the gene transfer efficiency of animal cells can be predicted easily and efficiently.

The present invention has been completed upon further research based on this finding, and provides, for example, the following method for predicting the gene transfer efficiency of animal cells.

[1] A method for predicting the gene transfer efficiency of animal cells, comprising:

(1) measuring the size of transgenic animal cells; and

(2) predicting the gene transfer efficiency based on values measured in step (1).

[2] The method according to [1], wherein in step (2), the distribution of cell sizes is used to predict the gene transfer efficiency of a cell population.

[3] The method according to [1] or [2], wherein in step (2), a parameter of the distribution of cell sizes is used to predict the gene transfer efficiency of the cell population.

[4] The method according to [3], wherein the parameter of the distribution of cell sizes is at least one selected from the group consisting of mean, median, mode, variance, kurtosis, and skewness.

[4a] The method according to [4], wherein the parameter of the distribution of cell sizes is mean or median.

[5] The method according to [2], wherein in step (2), the distribution of sizes of transfected animal cells is determined from the distribution of cell sizes to thereby predict the gene transfer efficiency of the cell population.

[6] The method according to any one of [1] to [5], wherein in step (2), in addition to the values measured in step (1), values measured at least at one stage before and after gene transfer are used to predict the gene transfer efficiency of the cell population.

[7] The method according to [1], wherein in step (2), changes in cell size before and after gene transfer are used to predict the gene transfer efficiency.

[8] The method according to any one of [1] to [7], wherein the cell size is cell diameter, volume, surface area, or area (indicating area when viewed in plan view).

[9] The method according to any one of [1] to [7], wherein forward scatter (FSC) values measured by a flow cytometer are used as the cell size values.

[10] The method according to [9], wherein in step (2), at least one selected from the group consisting of FSC pulse height, width, and area is used to predict the gene transfer efficiency of the cell population.

[11] The method according to any one of [1] to [10], wherein the animal cells are immune cells.

[12] The method according to [11], wherein the immune cells are T cells or NK cells.

[13] The method according to any one of [1] to [10], wherein the animal cells are epithelial cells.

[13a] The method according to [13], wherein the animal cells are liver cancer cells.

[13b] The method according to any one of [1] to [13a], wherein the animal cells are human cells.

[14] The method according to any one of [1] to [13b], wherein a nucleic acid to be introduced is a nucleic acid encoding a chimeric antigen receptor.

[15] The method according to any one of [1] to [14], wherein a size difference between transfected cells and non-transfected cells is 5% or more.

[16] The method according to [15], wherein the size difference between transfected cells and non-transfected cells is 5% or more for diameter and 5% or more for forward scatter (FSC) values measured by a flow cytometer.

[17] A method for producing a transgenic cell preparation, comprising;

(I) measuring the size of transgenic animal cells and measuring gene transfer efficiency based on measured values.

Advantageous Effects of Invention

According to the method for predicting gene transfer efficiency of the present invention, the gene transfer efficiency of cells can be measured with a simpler operation (e.g., the only operation required is automatic measurement using a measuring device) without the need for complicated steps, compared to conventional methods. Further, according to the method for predicting gene transfer efficiency of the present invention, the gene transfer efficiency of cells can be measured efficiently (e.g., in a short period of time of less than 5 minutes).

Brief Description of Drawings

Fig. 1 is a graph showing the results of Example 1. The vertical axis represents the CAR gene transfer efficiency (%) of CAR-T cells, and the horizontal axis represents the mean cell diameter (μm) (N=27).

Fig. 2 shows the results of Example 2. (A) is a graph showing the CAR gene transfer efficiency (%) of CAR-T cells and cell diameter distribution, and (B) is a graph showing the distribution of diameters (μm) of cells transduced with CAR gene determined by a mixed lognormal distribution model using the diameter (μm) of cells transduced with CAR gene measured by NC-200. The CAR-positive rate predicted by flow cytometry was 39.3%, and the CAR-positive rate determined from the mixed lognormal distribution model was 39.8%.

Fig. 3 shows the results of Example 3. (A) is a graph showing the cell diameter in each step of CAR-T production (the bottom, center line, and top of the box indicate the 25th, 50th, and 75th percentile values, respectively, for each calibration parameter), and (B) is a graph showing the CAR gene transfer efficiency (%) predicted by a machine learning model and the CAR gene transfer efficiency (%) predicted by flow cytometry (N=27).

Fig. 4 is a graph showing the distribution of diameters (μm) of cells transduced with CAR gene measured from the bright field in Example 4 (from above, CAR gene transfer efficiency (%): 0%, 47.6% (CAR-T cells), and 71.1% (CAR-T cells co-expressing IL-7 and CCL19)).

Fig. 5 shows the results of Example 5. (A) is a graph showing the size distribution of CAR-T cells measured by forward scatter (FSC) using flow cytometry, (B) to (E) are graphs showing the CAR gene transfer efficiency (%) of the "Neg," "Low," "Mid," and "High" portions in (A) measured by flow cytometry, and (F) is a graph showing the size distribution of CAR-T cells measured by FSC using flow cytometry (CAR gene transfer efficiency (%): 0% ("Untransduced"), 47.6%, and 71.1%).

Fig. 6 shows the results of Example 6. (A) is a graph showing the CAR gene transfer efficiency (%) of SK-HEP-1 (N=1), (B) is a graph showing the diameter (μm) of cells transduced with CAR gene (N=3477) and cells untransduced with CAR gene (N=3811) (p-values were calculated by a t-test) (the values shown in the graph are mean diameters), and (C) is a graph showing the distribution of diameters (μm) of cells untransduced with CAR gene and cells transduced with CAR gene (CAR gene transfer efficiency (%): 0% ("No gene transfer") and 18%).

Fig. 7 is a graph showing the CAR gene transfer efficiency (%) of CAR-T cells (horizontal axis), and the CAR gene transfer efficiency (%) predicted by regression analysis from the mean, median, mode, variance, skewness, or kurtosis of the diameter distribution (vertical axis) in Example 7.

Fig. 8 shows the results of Example 8. (A) is a graph showing the CAR gene transfer efficiency (%) and mean surface area ($\mu m^2$) of CAR-T cells, and (B) is a graph showing the CAR gene transfer efficiency (%) and mean volume ($\mu m^3$) of CAR-T cells.

Fig. 9 is a graph showing the mean diameter (μm) of cells before CAR gene transfer, untransduced cells, and transduced cells in Example 9 (N=1).

Fig. 10 is a graph showing the area ($\mu m^2$) of T cells and CAR-T cells in Example 10.

Fig. 11 shows a comparison of cell cycles between T cells and CAR-T cells in Example 11. (A) is a graph showing the CAR-positive rate, and (B) is a graph showing the results of cell cycle analysis measured by flow cytometry (the horizontal axis represents 7-AAD, and the vertical axis represents Cyclin A2).

Fig. 12 visualizes the gene expression profiles of starting materials and CAR-T cells after production in Example 12.

(A) visualizes CAR mRNA expression on a UMAP plot using a heatmap.

(B) shows the distribution of starting materials and CAR-T cells after production for each of three donors on the UMAP plot.

Fig. 13A shows the difference in mRNA expression profiles between T cells and CAR-T cells defined by the CAR mRNA expression in Example 12. (A) shows graphs plotted with the expression level of each mRNA on the horizontal axis for T cells and on the vertical axis for CAR-T cells.

Fig. 13B shows the difference in mRNA expression profiles between T cells and CAR-T cells defined by the CAR mRNA expression in Example 12. (B) shows Venn diagrams showing the number of mRNAs upregulated or downregulated in CAR-T cells relative to T cells in common with three donors.

Fig. 13C shows the difference in mRNA expression profiles between T cells and CAR-T cells defined by the CAR mRNA expression in Example 12. (C) is a table of mRNA expression variations common in three donors.

Fig. 13D shows the difference in mRNA expression profiles between T cells and CAR-T cells defined by the CAR mRNA expression in Example 12. (D) is a table showing the top 10 pathways with low p-values as a result of enrichment analysis performed on pathway-related gene groups registered in the Gene Ontology database or Reactome pathway database based on differentially expressed genes.

Fig. 14A shows the difference in mRNA expression profiles between T cells and CAR-T cells defined by the CAR protein expression in Example 13. (A) shows graphs plotted with the expression level of each mRNA on the horizontal axis for T cells and on the vertical axis for CAR-T cells.

Fig. 14B shows the difference in mRNA expression profiles between T cells and CAR-T cells defined by the CAR protein expression in Example 13. (B) shows Venn diagrams showing the number of mRNAs upregulated or downregulated in CAR-T cells relative to T cells in common with three donors.

Fig. 14C shows the difference in mRNA expression profiles between T cells and CAR-T cells defined by the CAR protein expression in Example 13. (C) is a table of mRNA expression variations common in three donors.

Fig. 14D shows the difference in mRNA expression profiles between T cells and CAR-T cells defined by the CAR protein expression in Example 13. (D) is a table showing the top 10 pathways with low p-values as a result of enrichment analysis performed on pathway-related gene groups registered in the Gene Ontology database or Reactome pathway database based on differentially expressed genes.

Fig. 14E shows the difference in mRNA expression profiles between T cells and CAR-T cells defined by the CAR protein expression in Example 13. (E) shows graphs showing the correlation between the CAR-T cell expression change ratio of each mRNA relative to T cells based on CAR mRNA, which is plotted on the horizontal axis, and the CAR-T cell expression change ratio of each mRNA relative to T cells based on CAR protein, which is plotted on the vertical axis.

Fig. 15A is a graph showing the results of phenotype analysis of T cells and CAR-T cells using a flow cytometer in Example 14. Regarding the ratio of cells with each phenotype, (A) shows phenotypes with a significant difference ($p<0.05$) as a result of a t-test performed on CAR-expressing cells and non-CAR-expressing cells.

Fig. 15B is a graph showing the results of phenotype analysis of T cells and CAR-T cells using a flow cytometer in Example 14. Regarding the ratio of cells with each phenotype, (B) shows the CD38-positive cell ratio and CD25-positive cell ratio, and (C) shows differences in the differentiation status of T cells. Tn: naive T cells, Tscm: stem cell memory T cells, Tcm:

central memory T cells, Tem: effector memory T cells, Teff:

effector T cells.

Fig. 16 shows the results of Example 15. (A) is a graph showing the fractionation of mCherry gene transfer γδ T cells by flow cytometry, (B) is a graph showing the FSC histograms of mCherry-negative cells and mCherry-positive cells, and (C) is a graph showing the median FSC of mCherry-negative cells and mCherry-positive cells (a t-test was performed on the median FSC) (N=4, error ranges are standard deviations).

Fig. 17 shows the results of Example 16. (A) is a graph showing the fractionation of CAR gene transfer γδ T cells by flow cytometry, and (B) is a graph showing the median FSC of CAR-negative cells and CAR-positive cells.

Fig. 18 shows the results of Example 17. (A) is a graph showing the fractionation of CAR gene transfer NK92 cells by flow cytometry, and (B) is a graph showing the median FSC of CAR-negative cells and CAR-positive cells (a t-test was performed on the median FSC) (N=4, error ranges are standard deviations).

Fig. 19 shows the results of Example 18. (A) is a graph showing the fractionation of CAR gene transfer NK cells by flow cytometry, (B) is a graph showing the FSC histograms of CAR-negative cells and CAR-positive cells, and (C) is a graph showing the median FSC of CAR-negative cells and CAR-positive cells (a t-test was performed on the median FSC) (N=4, error ranges are standard deviations).

Fig. 20 shows the results of Example 19. A graph showing the fractionation of CAR gene transfer T cells by flow cytometry in CAR and HLA-DR.

Description of Embodiments

Embodiments of the present invention are described in detail below.

The term "comprise(s)" or "comprising" means that although elements following these terms are included, the inclusion is not limited to the elements. Therefore, these terms suggest inclusion of elements following them, but do not suggest exclusion of any other elements. The term "consist(s) of" or "consisting of" means that any elements following these terms are included, and that the inclusion is limited to the elements. Therefore, the term "consist(s) of" or "consisting of" indicates that the listed elements are required or essential, and that there are substantially no other elements. The term "consist(s) essentially of" or "consisting essentially of" means that any elements following these terms are included, and that there is a limitation to other elements that do not affect the activity or action specified in the present disclosure for the above elements. Therefore, the term "consist(s) essentially of" or "consisting essentially of" indicates that the listed elements are required or essential, while other elements are optional, and may be or may not be present depending on whether they affect the activity or action of the listed elements.

In the present specification, "pluripotent stem cells" refer to embryonic stem cells (ES cells) and cells with similar pluripotency, i.e., cells with the potential to differentiate into various tissues (endoderm, mesoderm, and ectoderm) of a living body. Examples of cells with pluripotency similar to that of ES cells include induced pluripotent stem cells (also referred to as "iPS cells" in the present specification). If the pluripotent stem cells are ES cells or any cells derived from a human embryo, those cells may be produced by destroying the embryo or without destroying the embryo, and are preferably produced without destroying the embryo.

In the present specification, the term "cell population" refers to two or more cells of the same or different kind. The term "cell population" also refers to a mass of cells of the same or different kind.

The method for predicting the gene transfer efficiency of animal cells according to the present invention characteristically comprises:

(1) measuring the size of transgenic animal cells; and
(2) predicting the gene transfer efficiency based on values measured in step (1).

The term "gene transfer efficiency" in the present invention refers to the rate of transferring nucleic acids into cells, and is, for example, the percentage of cells into which nucleic acids are transferred among all cells, the nucleic acid intake amount of the cell population, or the transgene expression rate of the whole cell population. When the transgene is a CAR, it is also referred to as the "CAR-positive rate."

- Step (1)

In step (1), the size of transgenic animal cells is measured.

The type of animal cells is not particularly limited, and various animal cells can be widely used. Examples of the type of animal cells include splenocytes, neurons, glial cells, pancreatic β cells, bone marrow cells, mesangial cells, Langerhans cells, epidermal cells, epithelial cells, endothelial cells, fibroblasts, fibrocytes, muscle cells (e.g., skeletal muscle cells, cardiac myocytes, myoblasts, and satellite cells), adipocytes, immune cells (e.g., macrophages, T cells, B cells, natural killer cells (NK cells), mast cells, neutrophils, basophils, eosinophils, monocytes, and megakaryocytes), synovial cells, chondrocytes, bone cells, osteoblasts, osteoclasts, mammary cells, hepatocytes, stromal cells, egg cells, and sperm cells, as well as stem cells that can be induced to differentiate into these cells (including neural stem cells, hematopoietic stem cells, mesenchymal stem cells, dental pulp stem cells, iPS cells, ES cells, and other pluripotent stem cells), progenitor cells, blood cells, oocytes, and fertilized eggs. T cells include αβ T cells, γδ T cells, helper T cells, cytotoxic T cells, regulatory T cells, suppressor T cells, tumor-infiltrating T cells, memory T cells, naive T cells, NK T cells, TCR-T cells, STAR receptor T cells, CAR-T cells, and the like. Further, animal cells also include primary cells, the above cells produced by inducing the in vitro differentiation of the above stem cells (e.g., iPS cells), and the like. In addition, animal cells also include various cancer cells. Animal cells may be contained singly or in combination of two or more.

Organisms of origin of animal cells are not particularly limited, and examples of such organisms include mammals, such as humans, mice, rats, cows, horses, pigs, rabbits, dogs, cats, goats, monkeys, and chimpanzees. Preferred among these are humans.

Animal cells are animal cells into which an exogenous gene is introduced. The "exogenous gene" is a gene introduced from the outside into animal cells in order to express a desired protein, or monomer nucleotide, and can be suitably selected depending on the use of animal cells. The "transgenic animal cells" in the present invention include both animal cells that have actually been transfected with a gene as a result of an attempt of gene transfer, and animal cells that have not been transfected with a gene. Further, in the present invention, the "transfected (animal) cells" refer to (animal) cells that have actually been transfected with a gene. "Transgenic cells" and "transfected cells" may be used interchangeably, depending on the context.

The exogenous gene can be, for example, a gene for expressing a chimeric antigen receptor (CAR). The exogenous gene can be, for example, a gene for expressing a CAR and a gene for expressing a cytokine and/or a chemokine. As with general or known CARs, the CARs expressed by animal cells are basically configured such that peptides at sites of (i) an antigen recognition site that recognizes cell surface antigens of cancer cells (e.g., single-chain antibody, ligand, and peptide), (ii) a transmembrane region, and (iii) a signal transduction region that induces the activation of T cells, are linked via a spacer, as needed. Examples of other exogenous genes include T-cell receptors (TCRs), synthetic T-cell receptor and antigen receptor (STARs), chimeric T-cell antigen coupler (TAC) receptors, suicide genes (iCas9, HSV-TK, etc.), cytokines (interleukins, chemokines, etc.), and the like.

The means for introducing the exogenous gene into animal cells is not particularly limited, and various known or general means can be used. Typically, the exogenous gene is introduced into animal cells using an expression vector, and is expressed. The expression vector may be linear or cyclic, and may be a nonviral vector such as a plasmid, a viral vector, or a transposon vector.

The means for introducing the expression vector into animal cells can be made appropriate according to the embodiment. For example, the expression vector can be introduced into animal cells by a known method, such as a virus infection method, a calcium phosphate method, a lipofection method, a microinjection method, or an electroporation method. The expression vector can be prepared in a form suitable for use in each method by known means, or using commercially available kits as appropriate (according to the instructions thereof).

The expression vector can be introduced into animal cells by a virus infection method. Examples of viral vectors include retroviral vectors, lentiviral vectors, adenoviral vectors, and adeno-associated viral vectors. When using these viral vectors, a vector containing a desired exogenous gene and a packaging vector (plasmid) of each virus may be transfected into host cells using a corresponding commercially available kit to produce a recombinant virus, and then animal cells may be infected with the obtained recombinant virus.

When a plurality of exogenous genes are used, all of the exogenous genes may be contained in one expression vector, all of the exogenous genes may be separately contained in different expression vectors, or some of the plurality of exogenous genes may be contained in one expression vector, and the other genes may be separately contained in different expression vectors. When one expression vector contains a plurality of exogenous genes, the order in which those exogenous genes are arranged from the upstream side to the downstream side is not particularly limited.

The exogenous gene can be composed of a nucleic acid (polynucleotide) having a base sequence encoding the amino acid sequence of a desired protein or polypeptide. Those skilled in the art would be able to design and produce an expression vector capable of expressing a desired protein (polypeptide) in animal cells. The nucleic acids contained in the expression vector may be produced by chemically synthesizing DNA or may be produced (cloned) as cDNA.

In addition to the exogenous gene, the expression vector may contain sequences, such as promoter, terminator, enhancer, start codon, stop codon, polyadenylation signal, nuclear localization signal (NLS), and multi-cloning site (MCS), if necessary. The expression vector may further contain a nucleic acid (base sequence) encoding "functional genes," such as reporter genes (e.g., genes encoding various color fluorescent proteins), drug selection genes (e.g., kanamycin resistance gene, ampicillin resistance gene, and puromycin resistance gene), and suicide genes (e.g., genes encoding diphtheria A toxin, herpes simplex virus thymidine kinase (HSV-TK), carboxypeptidase G2 (CPG2), carboxylesterase (CA), cytosine deaminase (CD), cytochrome P450 (cyt-450), deoxycytidine kinase (dCK), nitroreductase (NR), purine nucleoside phosphorylase (PNP), thymidine phosphorylase (TP), varicella zoster virus thymidine kinase (VZV-TK), xanthine-guanine phosphoribosyltransferase (XGPRT), inducible caspase 9, etc.).

The "nucleic acid" may be any monomer nucleotide or any molecule obtained by polymerizing a nucleotide and a molecule having the same function as the nucleotide. Examples include RNA that is a polymer of ribonucleotide, DNA that is a polymer of deoxyribonucleotide, a polymer that is a mixture of ribonucleotide and deoxyribonucleotide, and a nucleotide polymer containing a nucleotide analog. The nucleic acid may also be a nucleotide polymer containing a nucleic acid derivative. The nucleic acid may be a single-stranded nucleic acid or a double-stranded nucleic acid. Double-stranded nucleic acids include a double-stranded nucleic acid in which one strand hybridizes to the other strand under stringent conditions.

The nucleotide analog may be any molecule as long as it is a molecule obtained by modifying ribonucleotide, deoxyribonucleotide, RNA, or DNA, for improvement of nuclease resistance, stabilization, increase in affinity with complementary strand nucleic acids, enhancement of cell permeability, or visualization, compared with RNA or DNA. The nucleotide analog may be a naturally occurring molecule or a non-natural molecule. Examples include sugar-modified nucleotide analogs (e.g., 2'-O-methylribose-substituted nucleotide analog, 2'-O-propylribose-substituted nucleotide analog, 2'-methoxyethoxyribose-substituted nucleotide analog, 2'-O-methoxyethylribose-substituted nucleotide analog, 2'-O-[2-(guanidium)ethyl]ribose-substituted nucleotide analog, 2'-fluororibose-substituted nucleotide analog, bridged nucleic acid (BNA), locked nucleic acid (LNA), ethylene-bridged nucleic acid (ENA), peptide nucleic acid (PNA), oxy-peptide nucleic acid (OPNA), and peptide ribonucleic acid (PRNA)), phosphodiester bond-modified nucleotide analogs (e.g., phosphorothioate bond-substituted nucleotide analog and N3'-P5' phosphoramidate bond-substituted nucleotide analog), and the like.

The nucleic acid derivative may be any molecule as long as it is a molecule obtained by adding another chemical substance to a nucleic acid, for improvement of nuclease resistance, stabilization, increase in affinity with complementary strand nucleic acids, enhancement of cell permeability, or visualization, compared with the nucleic acid. Specific examples include 5'-polyamine-adduct derivatives, cholesterol-adduct derivatives, steroid-adduct derivatives, bile acid-adduct derivatives, vitamin-adduct derivatives, Cy5-adduct derivatives, Cy3-adduct derivatives, 6-FAM-adduct derivatives, biotin-adduct derivatives, and the like.

The index used to show the "cell size" is not particularly limited as long as it can show the size of cells. Examples of the cell size include cell diameter (e.g., major and minor axes), radius, cell area and circumference length when viewed in plan view, cell volume and surface area, cell weight, sedimentation coefficient, and the like.

The method for measuring the cell size is not particularly limited as long as it can measure the cell size, and examples include methods using various commercially available measurement devices, visual measurement methods using microscopes, image analysis using microscopes, and the like. Examples of such devices for measuring the cell size include cell counting devices, cell counters, flow cytometers, Coulter counters, and the like.

Examples of cell counting devices (cell counters) include products of Chemometec (e.g., NC-200), Bio-Rad Laboratories (e.g., TC-20 Automated Cell Counter), Nova Biomedical (e.g., BioProfile FLEX and BioProfile FLEX2), Thermo Fisher Scientific (e.g., Countess 3 Automated Cell Counter), Beckman Coulter, Inc. (e.g., Vi-CELL XR Cell Viability Analyzer), Roche Diagnostic (e.g., Cedex HiRes automated cell analyzer), Nepa Gene (e.g., CASY Cell Counter & Analyzer), and the like. Cell counting devices and cell counters can measure the cell diameter. The measurement of the cell diameter can be performed according to a known method, for example, according to the manufacturer's manual of the cell counting device or cell counter.

Examples of flow cytometers include products of Beckman Coulter, Inc. (e.g., Gallios, Navios, Navios EX, CytoFLEX, CytoFLEX S, CytoFLEX LX, Cytomics FC 500, and DxH500), BD Biosciences (e.g., BD FACSCalibur™ flow cytometer, BD FACSCanto™ II flow cytometer, BD FACSVerse™ flow cytometer, BD FACSLyric™ flow cytometer, BD LSRFortessa™ flow cytometer, BD LSRFortessa™X-20 flow cytometer, and BD FACSymphony™ flow cytometer), Thermo Fisher Scientific (e.g., Attune flow cytometer), Agilent Technologies (e.g., Novocyte flow cytometer), Sartorius (e.g., iQue3 flow cytometer), Sony Corporation (e.g., SA3800 and SP6800Z), Sysmex Corporation (e.g., Multiparameter Automated Hematology Analyzer XN-550, Multiparameter Automated Hematology Analyzer XN-450, Multiparameter Automated Hematology Analyzer XN-350, Multiparameter Automated Hematology Analyzer XN-330, Multiparameter Automated Hematology Analyzer pocH™-80i, and Multiparameter Automated Hematology Analyzer XQ-320), and the like. Flow cytometry can measure the cell size based on forward scatter (FSC). The measurement of the cell size using a flow cytometer can be performed according to a known method, for example, according to the manufacturer's manual of the flow cytometer.

Examples of Coulter counters include products of Beckman Coulter, Inc. (e.g., Multisizer 3 and Multisizer 4e), Sysmex Corporation (e.g., Multiparameter Automated Hematology Analyzer XP-300, Particle Counter/Analyzer CDA-1000/1000B, Animal Multiparameter Automated Hematology Analyzer pocH™-100iV Diff/pocH™-100iV), Merck Millipore (e.g., Scepter 2.0 Cell Counter), Nihon Kohden (e.g., Automated Hematology Analyzer MEK-9200 Celltac G+, Automated Hematology Analyzer MEK-9100 Celltac G, Automated Hematology Analyzer MEK-7300 Celltac Es, Automated Hematology and Clinical Chemistry Analyzer MEK-1303 Celltac $\alpha$+, Automated Hematology Analyzer MEK-1303 Celltac $\alpha$, and Automated Hematology Analyzer MEK-6550 Celltac $\alpha$), and the like. Coulter counters can measure the cell volume and cell surface area. The measurement of the cell volume and cell surface area using a Coulter counter can be performed according to a known method, for example, according to the manufacturer's manual of the Coulter counter. The Coulter counter can also be used to measure the cell diameter.

- Step (2)

In step (2), the gene transfer efficiency is predicted based on values measured in step (1).

Since the correlation between the cell size and the gene transfer efficiency was confirmed in the Examples described below, the measured cell size values can be used to predict the gene transfer efficiency. In the present invention, the term "prediction" is used in the same sense as "measurement," and these terms can be used interchangeably.

In step (2), the distribution of cell sizes can be used to predict the gene transfer efficiency of the cell population. As an embodiment, parameters of the distribution of cell sizes are used to predict the gene transfer efficiency of the cell population. Such parameters of the distribution of cell sizes are not particularly limited, and examples include the mean, median, mode, variance (standard deviation), kurtosis, skewness, etc. of the values measured in step (1). The mean, median, mode, variance (standard deviation), and skewness are positively correlated with the gene transfer efficiency, and the kurtosis is negatively correlated with the gene transfer efficiency.

As the method for using cell size distribution parameters to predict the gene transfer efficiency of the cell population, for example, a calibration curve is created based on the gene transfer efficiency of cells whose gene transfer efficiency is known and cell size distribution parameters, and the calibration curve is used to determine the gene transfer efficiency from parameters obtained from the values measured in step (1). The calibration curve can be created by an ordinary method, such as the least squares method, using software or the like.

As another embodiment of step (2), the distribution of sizes of transfected animal cells is determined from the cell size distribution to predict the gene transfer efficiency of the cell population. For example, the gene transfer efficiency of the cell population is predicted by using a mixed lognormal distribution model to determine the ratio of two populations, a transfected cell population and a non-transfected cell population, to the whole cell population. Specifically, each parameter of the mixed lognormal distribution model is estimated by fitting a mixed normal distribution to the log-transformed data of the values measured in step (1) using the maximum likelihood method, and the gene transfer efficiency of the cell population is determined (for details, see the Examples described below). Such a calculation process can be performed by using known software, such as R, SAS, SPSS, or JMP.

As still another embodiment of step (2), the gene transfer efficiency of the cell population is predicted by using, in addition to the values measured in step (1), values measured at least at one stage before and after gene transfer to predict the gene transfer efficiency of the cell population. That is, the size of animal cells is measured in multiple steps for producing transgenic animal cells, and animal cell size values at multiple time points, not just one, are used to predict the gene transfer efficiency. Using cell sizes at multiple time points in this way enables robust analysis. When predicting the gene transfer efficiency using animal cell size values at multiple time points, the number of multiple time points is, for example, 2, 3, 4, 5, 6, 7, 8, 9, or 10. Specific examples of multiple time points include raw materials, after activation of the cells, after gene transfer into the cells, after expanded culture after gene transfer, and the like. The animal cell size values used herein are preferably the cell size distribution parameters described above. The gene transfer efficiency of the cell population can be predicted by, for example, using a machine learning model. That is, training data with known gene transfer efficiency are given to a prediction model to learn the data to create a trained prediction model, and then animal cell size values measured at multiple time points are input into the learned prediction model to predict the gene transfer efficiency. The machine learning model may be one capable of performing regression analysis, and examples include lasso regression, ridge regression, elastic-net regression, principal component regression, partial least squares regression, random forest, gradient boosting decision tree, neural network, deep learning, support vector machine, and the like.

As still another embodiment of steps (1) and (2), the gene transfer efficiency of the cell population is predicted by performing the cell size measurement in step (1) by measuring FSC with a flow cytometer, thereby using FSC pulse height, width, and area in step (2) for the prediction of the gene transfer efficiency of the cell population. That is, at least one selected from the group consisting of FSC pulse height, width, and area measured in the individual cells is used to predict the gene transfer efficiency. Using multiple values in this way enables robust analysis. The gene transfer efficiency of the cell population can be predicted by, for example, using a machine learning model. That is, training data with known gene transfer efficiency are given to a prediction model to learn the data to create a trained prediction model, and then animal cell size values are input into the learned prediction model to predict the gene transfer efficiency. As a specific example, a trained prediction model is created using the data determining the presence or absence of gene transfer in the individual cells as correct labels, and the measurement values of FSC pulse height, width, and area as learning data, and then the measured values of FSC pulse height, width, and area of the individual cells are input into the trained prediction model to predict the gene transfer efficiency. As the learning data, FSC distribution parameters of the cell population may be used in addition to measured values of FSC pulse height, width, and area of the individual cells. Since the prediction gives the probability of being classified as transfected cells, the presence or absence of gene transfer in the individual cells may be determined by a specific threshold (e.g., 50%), and the gene transfer rate of the cell population may be determined. Alternatively, the mean probability of being classified as transfected cells for the individual cells may be used as the gene transfer efficiency of the cell population. The machine learning model may be one capable of performing regression analysis, and examples include lasso regression, ridge regression, elastic-net regression, principal component regression, partial least squares regression, random forest, gradient boosting decision tree, neural network, deep learning, support vector machine, and the like.

In step (2), changes in cell size before and after gene transfer can also be used to predict the gene transfer efficiency. The Examples described below show that the cell size changes before and after gene transfer, and increases. Therefore, even for a single cell, it is possible to predict whether gene transfer is present by measuring and comparing the cell size before and after gene transfer.

The difference in size (particularly diameter and forward scatter (FSC) values measured by a flow cytometer) between transfected cells (animal cells actually transfected with a gene) and non-transfected cells (animal cells not transfected with a gene) is such that, particularly among the size distribution parameters of the transfected cell population and non-transfected cell population, one or more of mean, median, and mode (particularly mean or median) are, for example, 5% or more, 6% or more, 7% or more, 8% or more, 9% or more, or 10% or more. The size of transfected cells is larger than that of non-transfected cells. When the size is volume, the difference in size between transfected cells and non-transfected cells is such that, particularly among the size distribution parameters of the transfected cell population and non-transfected cell population, one or more of mean, median, and mode (particularly mean or median) are 5% or more, 6% or more, 7% or more, 8% or more, 9% or more, 10% or more, 11% or more, 12% or more, 13% or more, 14% or more, or 15% or more. When the size is surface area or area, the difference in size between transfected cells and non-transfected cells is such that, particularly among the size distribution parameters of the transfected cell population and non-transfected cell population, one or more of mean, median, and mode (particularly mean or median) are 5% or more, 6% or more, 7% or more, 8% or more, 9% or more, or 10% or more.

The method for producing a transgenic cell preparation of the present invention (also referred to simply as "the production method of the present invention" in the present specification) characteristically comprises:

(I) measuring the size of transgenic animal cells, and predicting the gene transfer efficiency based on the measured values.

The production method of the present invention may further comprise:
(II) introducing a gene into an animal cell.

Steps (I) and (II) of the production method of the present invention can be carried out by the method described above. The cell preparation produced by the production method of the present invention (hereinafter also referred to as "the cell preparation of the present invention") is preferably produced as a parenteral preparation by mixing an effective amount of transgenic animal cells with a pharmaceutically acceptable carrier according to known means (e.g., the methods described in the Japanese Pharmacopoeia). The cell preparation of the present invention is preferably produced as a parenteral preparation, such as injection, suspension, or infusion. Examples of parenteral administration methods include intravenous, intraarterial, intramuscular, intraperitoneal, or subcutaneous administration. Examples of the pharmaceutically acceptable carrier include solvents, bases, diluents, excipients, soothing agents, buffers, preservatives, stabilizers, suspensions, isotonic agents, surfactants, solubilizing agents, and the like.

The dose of the cell preparation of the present invention can be suitably determined depending on various conditions, such as patient's body weight, age, sex, and symptoms. The cell preparation of the present invention may be administered once or several times. The cell preparation of the present invention can have a known form suitable for parenteral administration, such as injection or infusion. Further, the cell preparation of the present invention may contain physiological saline, phosphate buffered saline (PBS), medium, and the like in order to stably maintain the cells. Examples of the medium include RPMI, AIM-V, X-VIVO10, and other media, but are not limited thereto. In addition, pharmaceutically acceptable carriers (e.g., human serum albumin), preservatives, and the like may be added to the cell preparation for the purpose of stabilization. The cell preparation of the present invention is applied to mammals, including humans.

In contrast to conventional methods using flow cytometry etc., which require manual work, the method for predicting gene transfer efficiency of the present invention does not require such complicated steps and makes it possible to measure the gene transfer efficiency of cells with a simple operation, such as automatic measurement using a measuring device. Further, the method for predicting gene transfer efficiency of the present invention makes it possible to measure the gene transfer efficiency of cells efficiently, for example, in a short period of time of less than 5 minutes, whereas conventional methods take 6 to 7 hours. In addition, the method of the present invention and the cell type are not particularly limited, and the method can be applied to various cells. It is expected that the method of the present invention will be applied to gene therapies, such as CAR-T cell therapy.

As used in the present specification and claims, unless otherwise required by context, singular terms shall include pluralities and plural terms shall include the singular. Thus, singular articles (e.g., "a," "an," "the," and the like in the case of English) should also be understood as encompassing the concepts thereof in the plural form unless specifically noted otherwise.

## Examples

Examples are provided below in order to explain the present invention in more detail. However, the present invention is not limited to these Examples.

### Method

#### Medium

CAR-T cell culture medium: 2.6% OpTmizer Expansion Basal Supplement (Thermo Fisher Scientific), 1% L-Glutamine (Thermo Fisher Scientific), 1% Streptomycin, and 2% CTS Immune Cell SR (Thermo Fisher Scientific) were added to OpTmizer CTS T-Cell Expansion basal medium (Thermo Fisher Scientific) to produce a basal medium. MACS GMP IL-2 (Miltenyi Biotec) was added thereto at 20 IU/mL or 40 IU/mL.

SK-HEP-1 cell culture medium: 10% FBS (Biosera), 1% Non-essential amino acids (FUJIFILM Wako Pure Chemical Corporation), 1% Penicillin-Streptomycin solution (FUJIFILM Wako Pure Chemical Corporation), and 1 mM Sodium pyruvate (FUJIFILM Wako Pure Chemical Corporation) were added to MEM, L-Gln (+) (Thermo Fisher Scientific) for production.

NK92 cell culture medium: 20% FBS (Biosera) was added to RPMI 1640 Media (Thermo Fisher Scientific), and MACS GMP IL-2 (Miltenyi Biotec) was added at 200 IU/mL for production.

#### Gene transfer into T cells

After thawing PBMC (Precision for Medicine), Leukopak (Hemacare), or γδ T cells (Hemacare), the cells were diluted in the CAR-T cell culture medium to a con-

centration of $2.0 \times 10^6$ cells/mL or less (raw materials before production). The cells were seeded in a culture bag so that the ratio of cell suspension to MACS GMP T-Cell TransACT (Miltenyi Biotec) was 17.5:1, and cultured for more than 36 hours to less than 48 hours (cell activation step). The activated cells were diluted in the culture medium using the LOVO Cell Processing System (Fresenius Kabi) or a centrifuge, and seeded at $6.07 \times 10^5$ cells/cm$^2$ or less in a culture bag that was pre-coated with Retronectin (trademark) (Takara Bio Inc.) and retrovirus with CAR gene, with CAR gene, IL-7 gene, and CCL19 gene, or with mCherry gene, and cultured until the next day (gene transfer step). The cells were seeded at $2.2 \times 10^6$ cells/cm$^2$ or less in a culture bottle (G-REX, Wilson Wolf), and cultured for 3 to 7 days, thereby producing CAR-T cells (final product). The CAR gene used has a base sequence represented by SEQ ID NO: 1, the CAR gene, IL-7 gene, and CCL19 gene used have a base sequence represented by SEQ ID NO: 2, and the mCherry gene used has a base sequence represented by SEQ ID NO: 3 (the same applies to the following case of SK-HEP-1 cells).

CAR gene transfer into SK-HEP-1 cells

SK-HEP-1 cells (ATCC) were diluted in the SK-HEP-1 cell culture medium to a concentration of $2.0 \times 10^6$ cells/mL or less, seeded at $6.07 \times 10^5$ cells/cm$^2$ or less on a culture plate that was pre-coated with Retronectin (Takara Bio Inc.) and retrovirus with CAR gene, and cultured for 4 days, thereby introducing the CAR gene into the SK-HEP-1 cells.

Production of CAR-NK cells

After thawing NK-92 (ATCC), which is a cell line of NK cells, the cells were cultured in the NK92 cell culture medium. After culture, the cell suspension was seeded at $6.07 \times 10^5$ cells/cm$^2$ or less in a culture bag that was pre-coated with Retronectin (trademark) (Takara Bio Inc.) and retrovirus with CAR gene, thereby producing CAR-NK cells.

Primary cultured CAR-NK cells were produced using PBMC (Precision for Medicine) and BINKIT (trademark) NK cell expansion kit (Biotherapy Institute of Japan, Inc.) according to the manual.

Measurement of cell diameter using NC-200

The cell diameter was measured using an NC-200 cell counter (Chemometec) according to the manual. The cell diameter was analyzed with fluorescent dyes, acridine orange and DAPI.

Measurement of cell diameter using TC-20

The cell diameter was measured using the TC-20 Automated Cell Counter (BIO-RAD Laboratories) according to the manual. The cell diameter was analyzed in the bright field.

Measurement of size and gene transfer efficiency using flow cytometry

Using a BD FACS Canto II flow cytometer (BD Biosciences), gene transfer efficiency was measured using CAR antigen or mCherry fluorescence. The cell size was quantified by FSC.

Cell distribution analysis using mixed lognormal distribution model

Using statistical analysis software R, each parameter of a mixed lognormal distribution model was estimated by fitting a mixed normal distribution to log-transformed data of individual cell diameters or FSC measured with the NC-200 cell counter or by flow cytometry using the maximum likelihood method. The probability density function of the two-group mixed normal distribution is represented as follows:

$$p(x) = \lambda_1 N(x|\mu_1, \sigma_1) + \lambda_2 N(x|\mu_2, \sigma_2)$$

wherein x is the natural logarithm of each measured cell diameter,

$$N(x|\mu, \sigma)$$

represents the normal distribution of mean $\mu$ and variance $\sigma$, $\lambda$ is a weighting factor in the mixture distribution, and $\lambda_1+\lambda_2=1$. Each parameter was estimated by maximum likelihood using the EM algorithm so that the following log likelihood function of the probability density function p(x) was maximized:

$$\ln L(\lambda, \mu, \sigma|x)$$

Since the ratios of the two populations to the total cell population were $\lambda_1$ and $\lambda_2$, the weighting factor $\lambda$ of the larger population of $\mu_1$ and $\mu_2$ was estimated as the ratio of target transfected cells.

Prediction of gene transfer efficiency using machine learning model

Using the programming language Python in Jupyter Notebook, which is an integrated development environment that runs on a web browser, a machine learning model was constructed by the random forest algorithm to predict the CAR gene transfer efficiency (predicted CAR%) from the mean cell diameter during the production step. The cell diameter data during the production step were obtained from four points: raw materials before production, after activation of the cells, after gene transfer into the cells, and the final product. A prediction model was constructed using 21 out of 27 lots of prototypes as training data, and 6 lots were used as testing data and fitted to the model.

Measurement of cell volume and surface area using electrical sensing zone method

The cell volume and surface area were measured using a Multisizer 4e (Beckman Coulter, Inc.) according to the manual. The cell volume was analyzed by the Coulter principle, which is known as the electrical sensing zone method. The cell surface area was calculated based on the assumption that the surface area would be equivalent to the surface area obtained from a complete sphere, which is identical to each cell.

Quantification of cell area (I)

After antibody staining with CAR antibody, images were obtained using a Nanozoomer (Hamamatsu Photonics K.K.), and the area was evaluated using the image analysis software Halo (Indica Labs).

Quantification of cell area (II)

The cells were stained with CAR antibody, Cyclin A2 antibody (Beckman Coulter, Inc.), and 7-AAD (Beckman Coulter, Inc.), and then quantified by flow cytometry.

Gene expression analysis by scRNA-seq and Total-seq

The gene expression analysis by scRNAseq and Total-seq was performed in accordance with procedures (1) to (4).

(1) Frozen CAR-T cells and their starting materials, T cells, were thawed, blocked with Human TruStain FcX™ Fc Blocking reagent (BioLegend), and then stained with PE-labeled CAR antibody. Thereafter, they were stained with TotalSeq-C anti-PE antibody (BioLegend). (2) Using Chromium Next GEM Single Cell 5' Kit of 10X Genomics, one cell was isolated and reverse-transcribed from the antibody-stained sample. A library of gene expression, feature barcoding derived from TotalSeq-C antibody, and TCRs was prepared from the obtained cDNA. (3) Sequencing was performed using a next-generation sequencer HiSeq of Illumina. (4) Mapping and expression quantification were performed using Cell Ranger (10X Genomics) from the FASTQ files obtained by sequencing. The reference data used for mapping were created by adding the LTR region of the CAR plasmid used in gene transfer to the human genome library GRCh38. The mapped mRNA expression data and protein expression data were quality-controlled using R v3.6.3 and Seurat v3.2.3 to remove the gene expression data of cells with a mitochondrial gene rate of 10% or more, or with less than 200 genes detected, from the subsequent analysis targets. In addition, simulations were carried out within the sample using DoubletFinder v2.0.3, and gene expression data of cells that were assumed to be doublets were also removed from the subsequent analysis targets. The quality-controlled expression data were used for the analysis of gene expression profiles in Cell Ranger.

Phenotype analysis using flow cytometer

Measurements were performed by FACS symphony (BD Biosciences) using the following: CAR antibody, CD3 antibody (BD Biosciences), CD4 antibody (BD Biosciences), CD8 antibody (BD Biosciences), CD45RA antibody (BD Biosciences), CD45RO antibody (BD Biosciences), CD197 antibody (BD Biosciences), CD62L antibody (BD Biosciences), CD95 antibody (BD Biosciences), CD27 antibody (BD Biosciences), CD127 antibody (BD Biosciences), CD25 antibody (BD Biosciences), CD38 antibody (BD Biosciences), CD69 antibody (BD Biosciences), CD279 antibody (BD Biosciences), CD223 (BioLegend), CD366 antibody (BD Biosciences), CD152 antibody (BD Biosciences), CD28 antibody (BD Biosciences), KLRG1 antibody (BioLegend), CD57 antibody (BD Biosciences), and HLA-DR antibody (BioLegend).

<u>Example 1: Correlation between CAR gene transfer efficiency and</u>

<u>cell diameter of CAR-T cells co-expressing IL-7 and CCL19</u>

After CAR-T cells co-expressing IL-7 and CCL19 (T cells transduced with the CAR gene, IL-7 gene, and CCL19 gene described above) were produced, the diameter of the final cell product was measured by the acridine orange method using NC-200 (11 cancer patients, 10 healthy subjects, a total of 27 samples). In addition, the CAR gene transfer efficiency was measured by flow cytometry. The results are shown in Fig. 1. As a result, it was confirmed that the mean cell diameter was correlated with the CAR transfer rate (R = 0.77).

<u>Example 2: Prediction of gene transfer efficiency from cell</u>

<u>diameter distribution</u>

The diameter of CAR-T cells and CAR-T cells co-expressing IL-7 and CCL19 was measured using NC-200, a mixed lognormal distribution model consisting of two populations with different cell diameters was fitted to the distribution by maximum likelihood estimation, and the ratio of each population was calculated. In addition, the CAR gene transfer efficiency was measured by flow cytometry. The results are shown in Fig. 2. The distribution of cell diameters varied depending on the CAR gene transfer efficiency (Fig. 2A), and the ratio of the large-diameter cell population (39.8%) calculated using the mixed lognormal distribution model was very close to the CAR gene transfer efficiency (39.3%) measured by flow cytometry (Fig. 2B).

Example 3: Prediction of gene transfer efficiency using machine

learning model

The diameter was measured using NC-200 at the time periods of four steps: raw materials, after T cell activation, after gene transfer, and final product (CAR-T cells), in the production of CAR-T cells co-expressing IL-7 and CCL19, and the CAR gene transfer efficiency was predicted using a machine learning algorithm from the mean diameter data in the four steps. The results are shown in Fig. 3. As a result, it was confirmed that the cell diameter increased after gene transfer (Fig. 3A). It was also confirmed that the CAR gene transfer efficiency predicted from the diameter data in the four steps using the machine learning algorithm was correlated with the CAR gene transfer efficiency measured by flow cytometry in a conventional method (Fig. 3B).

Example 4: Measurement of cell diameter in bright field

The cell diameter was measured using acridine orange in Examples 1 and 2; however, in order to confirm whether the diameter increased according to the CAR gene transfer efficiency also in the bright field used for microscope observations etc., the diameter of CAR-T cells and CAR-T cells co-expressing IL-7 and CCL19 was measured using TC-20 from the bright field. The results are shown in Fig. 4. As a result, the diameter measured from the bright field increased as the CAR transfer rate increased.

Example 5: Measurement of cell size by flow cytometry

Using flow cytometry, the size of CAR-T cells and CAR-T cells co-expressing IL-7 and CCL19 was measured by forward scatter (FSC), and it was confirmed whether this could predict the CAR gene transfer efficiency. The maximum diameter is measured by FSC. The results are shown in Fig. 5. First, in order to perform a detailed analysis, it was confirmed whether FSC was changed in the CAR transfected cells. As a result, as shown in Figs. 5A to 5E, it was confirmed that FSC increased in the CAR-positive cells; that is, the cells became larger. In addition, the CAR gene transfer efficiency was predicted in detail from FSC distribution in the same manner as in Example 2. As a result, the cells whose CAR gene transfer efficiency was predicted to be 41.9% and 74.3% from the size measured by FSC showed very close values of 47.6% and 71.1%, respectively, for the CAR gene transfer efficiency as measured by flow cytometry in a conventional method (Fig. 5F).

Example 6: Prediction of gene transfer efficiency using SK-HEP-1

cells

Using the hepatocellular carcinoma cell line SK-HEP-1, it was confirmed whether the CAR gene transfer used in Example 2 would increase the cell size, regardless of the type of cells for transfer (i.e., other than T cells). The cell diameter was measured by NC-200. The results are shown in Fig. 6. As shown in Figs. 6B and 6C, it was confirmed that the cell diameter significantly increased in the CAR transfected cells.

Example 7: Comparison of cell size indices (basic statistics)

It was evaluated whether the CAR transfer rate could be predicted from indices other than the mean cell diameter in the same manner as in Example 1. The results are shown in Fig. 7. As a result, as the CAR positivity rate increased, the median, mode, variance, and skewness of the diameter distribution increased and the kurtosis decreased. Therefore, it was suggested that the CAR transfer rate could be predicted from all of these indexes. It was found that the transduced cells not only grew larger, but also showed greater variation in size.

Example 8: Measurement of cell volume and surface area using

Coulter counter

In order to evaluate whether the CAR transfer rate could be predicted from indices other than the cell diameter, the CAR-T cells described in Example 2 were used to measure the cell volume and surface area using the electrical sensing zone method. The results are shown in Fig. 8. As a result, it was confirmed that prediction was also possible from the volume (Fig. 8A) and surface area (Fig. 8B) in the same manner as for the diameter.

Example 9: Comparison of cell size between before and after gene

transfer

CAR-T cells co-expressing IL-7 and CCL19 were produced, and the cell diameter was measured before gene transfer and after production of the CAR-T cells by the acridine orange method using NC-200. The results are shown in Fig. 9. It was confirmed that the CAR-T cells were larger in the gene transfer group compared to the cell population without gene transfer before and after gene transfer.

Example 10: Comparison of area (μ$m^2$) between T cells and CAR-T

cells

In order to confirm whether the area was increased by CAR gene transfer, antibody staining was performed using CAR antibody after production of CAR-T cells, and the area of T cells and CAR-T cells was quantified. The results are shown in Fig. 10. As a result, the area was increased in the CAR-T cells.

Example 11: Cell cycle comparison between T cells and CAR-T cells

It is known that the cell size changes during cell proliferation and increases during the proliferation phase. Therefore, cell cycle analysis was performed by flow cytometry. The results are shown in Fig. 11. After CAR-T cells were produced, CAR-positive cells were fractionated using CAR antibody (Fig. 11A), and the cell cycle therein was confirmed (Fig. 11B). As a result, many of the T cells transduced with CAR gene were in the proliferation phases, S_G2 and M phases. This also demonstrated that the size was changed by the CAR gene transfer.

Example 12: Comparison of mRNA expression profiles between T cells

and CAR-T cells defined by CAR mRNA expression

In order to further examine the differences in properties between CAR-T cells and T cells, scRNA-seq analysis was performed. The quality-controlled scRNA-seq data were scaled and merged. The merged single-cell gene expression profiles were visualized by UMAP plotting. The results are shown in Fig. 12. The population to be analyzed in the future was confirmed by the expression of the target gene (Fig. 12A). Since CAR mRNA-expressing cells are widely distributed among CAR-T cells, CAR gene transfer is considered to occur relatively randomly (Fig. 12B). That is, it was confirmed that the differences in mRNA profiles between CAR-T cells and T cells were caused by the CAR gene transfer.

In addition, the samples were classified into a CAR expressed cluster and a CAR non-expressed cluster according to the presence or absence of gene expression of CAR mRNA in each sample, and the variation of gene expression between the clusters was analyzed. The results are shown in Fig. 13. Using Seurat v3.2.3, differentially expressed genes were detected based on the criterion that the expression change ratio was more than 1.5 times or less than 2/3 times, and the p-value obtained by Wilcoxon's rank sum test was less than 0.05 (Figs. 13A to 13C). Further, for the differentially expressed genes obtained by gene expression variation analysis, the overlap in biological functions and pathways was evaluated by enrichment analysis. Pathway-related gene groups registered in the Gene Ontology database and Reactome pathway database were subjected to enrichment analysis using clusterProfiler v3.14.3, and pathways that satisfied the significance level with a p-value of less than 0.05 were extracted. As a result, it was found that the pathway involved in T-cell activation significantly changed between CAR-T cells and T cells (Fig. 13D). Since T cells are known to increase in size upon activation, it is expected from gene expression that they become larger as well.

Example 13: Comparison of mRNA expression profiles between T cells

and CAR-T cells defined by CAR protein expression

In order to further examine the differences in properties between CAR-T cells and T cells, total-seq analysis was performed. As in Example 12, samples were classified into a CAR expressed cluster and a CAR non-expressed cluster according to the presence or absence of gene expression of CAR protein in each sample, and the variation of gene expression between the clusters was analyzed. The results are shown in Fig. 14. Using Seurat v3.2.3, differentially expressed genes were detected based on the criterion that the expression change ratio was more than 1.5 times or less than 2/3 times, and the p-value obtained by Wilcoxon's rank sum test was less than 0.05 (Figs. 14A to 14C). Further, for the differentially expressed genes obtained by gene expression variation analysis, the overlap in biological functions and pathways was evaluated by enrichment analysis. Pathway-related gene groups registered in the Gene Ontology database and Reactome pathway database were subjected to enrichment analysis using clusterProfiler v3.14.3, and pathways that satisfied the significance level with a p-value of less than 0.05 were extracted. As a result, it was found that the pathway involved in T-cell activation significantly changed between CAR-T cells and T cells (Fig. 14D). These results were consistent with those of Example 12. Further, as shown in Fig. 14E, a strong correlation was confirmed between the mRNA expression change ratio of CAR-T cells to T cells based on CAR mRNA and the expression change ratio based on the CAR protein.

Example 14: Phenotype comparison between T cells and CAR-T cells

using flow cytometer

For CAR-T cells derived from four different donors, phenotype analysis was performed by flow cytometry using the antibodies against biomarkers with general phenotypes described in "Phenotype analysis using flow cytometer." Then, using FlowJo software (BD Biosciences), the T-cell population and CAR-T cell population were defined by gating, and the expression of biomarkers with the phenotypes was comprehensively gated within them. A t-test was performed on the ratio of cells with each phenotype contained in each gate between CAR-expressing cells and non-CAR-expressing cells, and phenotypes with significant differences ($p<0.05$) were shown. The results are shown in Fig. 15.

As a result, the CD25-positive cell rate was higher and the CD38-positive cell rate was lower in the CAR-expressing cells compared to non-CAR-expressing cells. Since CD25 is a marker associated with activation, it is considered that the activation signal is also strong in the CAR-expressing cells, as in Examples 12 and 13, and therefore the size is larger. CD38 is also known as an activation marker, but it is also known to be highly expressed in exhausted T cells during late differentiation (Henning, Amanda N et al. Nature Reviews Immunology, vol. 18, 5 (2018): 340-356); thus, it cannot be treated as an activation marker in general. Moreover, the higher ratio of both CD45RA-positive and CD45RO-positive cells in the CAR-expressing cells suggests a higher ratio of stem cell memory T cells. Since stem cell memory T cells are generally known to have higher proliferative capacity than effector T cells or other phenotype T cells, it is considered that the proliferative capacity is also higher and the size is larger in the CAR-expressing cells as in Example 11. These trends were similar for CD4-positive cells and CD8-positive cells.

<u>Example 15: Changes in size of γδ T cells transduced with mCherry</u>

<u>gene</u>

After γδ T cells transduced with mCherry gene were produced, FSC was compared between mCherry-negative cells and mCherry-positive cells (Fig. 16A). As a result, FSC was increased in the mCherry-positive cells, and the γδ T cells were increased in size by mCherry transfer (Figs. 16B and 16C).

<u>Example 16: Changes in size of γδ T cells transduced with CAR gene</u>

After γδ T cells transduced with CAR gene were produced, FSC was compared between CAR-negative cells and CAR-positive cells (Fig. 17A). As a result, FSC was increased in the CAR-positive cells, and the γδ T cells were increased in size by CAR gene transfer (Fig. 17B).

<u>Example 17: Changes in size of CAR-NK cells (NK92 cells)</u>

After CAR-NK cells transduced with CAR gene were produced from NK92 cells, FSC was compared between CAR-negative cells and CAR-positive cells (Fig. 18A). As a result, FSC was increased in the CAR-positive cells, and the NK92 cells were increased in size by CAR gene transfer (Fig. 18B).

<u>Example 18: Changes in size of CAR-NK cells (primary cultured NK cells)</u>

After CAR-NK cells transduced with CAR gene were produced from primary cultured NK cells, FSC was compared between CAR-negative cells and CAR-positive cells. As a result, FSC was increased in the CAR-positive cells, and NK cells were increased in size by CAR gene transfer (Figs. 19A to 19C).

<u>Example 19: Promotion of activation by CAR introduction</u>

Since a tendency to promote activation was observed in Examples 12 and 13, it was confirmed whether activation was promoted in CAR-positive cells. As a result, the expression of HLA-DR, which is an activation marker, was increased in the CAR-positive cells, confirming that CARs promoted the activation (Fig. 20).

The present application is based on Japanese Patent Application No. 2021-209987 filed on December 23, 2021 and Japanese Patent Application No. 2022-158190 filed on September 30, 2022 in Japan, the entire contents of which are incorporated herein.

Sequence Listing

**Claims**

1. A method for predicting the gene transfer efficiency of animal cells, comprising:

    (1) measuring the size of transgenic animal cells; and
    (2) predicting the gene transfer efficiency based on values measured in step (1).

2. The method according to claim 1, wherein in step (2), the distribution of cell sizes is used to predict the gene transfer efficiency of a cell population.

3. The method according to claim 1, wherein in step (2), a parameter of the distribution of cell sizes is used to predict the gene transfer efficiency of the cell population.

4. The method according to claim 3, wherein the parameter of the distribution of cell sizes is at least one selected from the group consisting of mean, median, mode, variance, kurtosis, and skewness.

5. The method according to claim 2, wherein in step (2), the distribution of sizes of transfected animal cells is determined from the distribution of cell sizes to thereby predict the gene transfer efficiency of the cell population.

**6.** The method according to claim 1, wherein in step (2), in addition to the values measured in step (1), values measured at least at one stage before and after gene transfer are used to predict the gene transfer efficiency of the cell population.

**7.** The method according to claim 1, wherein in step (2), changes in cell size before and after gene transfer are used to predict the gene transfer efficiency.

**8.** The method according to claim 1, wherein the cell size is cell diameter, volume, surface area, or area.

**9.** The method according to claim 1, wherein forward scatter (FSC) values measured by a flow cytometer are used as the cell size values.

**10.** The method according to claim 9, wherein in step (2), at least one selected from the group consisting of FSC pulse height, width, and area is used to predict the gene transfer efficiency of the cell population.

**11.** The method according to claim 1, wherein the animal cells are immune cells.

**12.** The method according to claim 11, wherein the immune cells are T cells or NK cells.

**13.** The method according to claim 1, wherein the animal cells are epithelial cells.

**14.** The method according to claim 1, wherein a nucleic acid to be introduced is a nucleic acid encoding a chimeric antigen receptor.

**15.** The method according to claim 1, wherein a size difference between transfected cells and non-transfected cells is 5% or more.

**16.** The method according to claim 15, wherein the size difference between transfected cells and non-transfected cells is 5% or more for diameter and 5% or more for forward scatter (FSC) values measured by a flow cytometer.

**17.** A method for producing a transgenic cell preparation, comprising;

(I) measuring the size of transgenic animal cells and measuring the gene transfer efficiency based on measured values.

Fig. 1

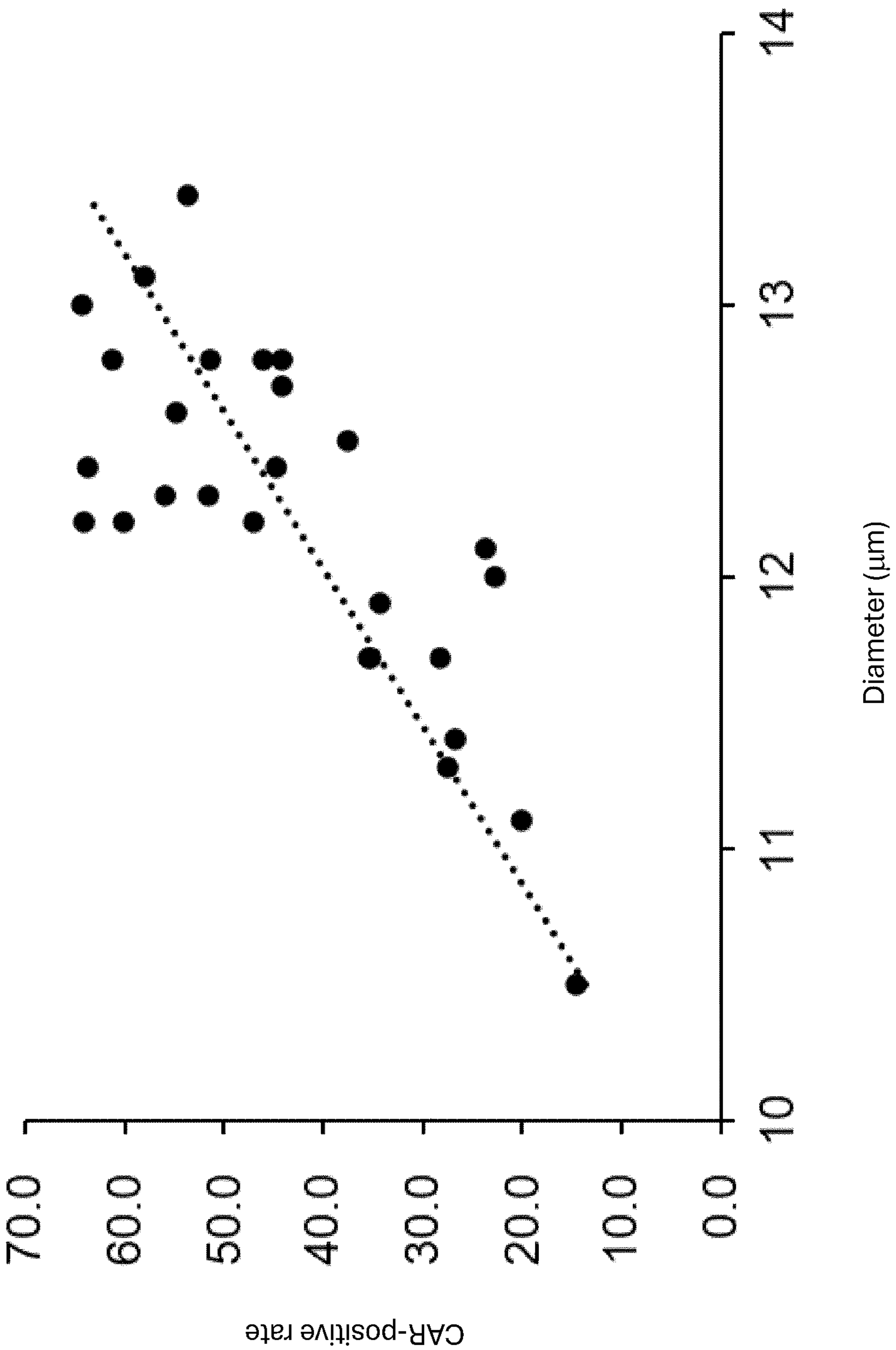
CAR-positive rate
70.0
60.0
50.0
40.0
30.0
20.0
10.0
0.0
10
11
12
13
14
Diameter (μm)

Fig. 2

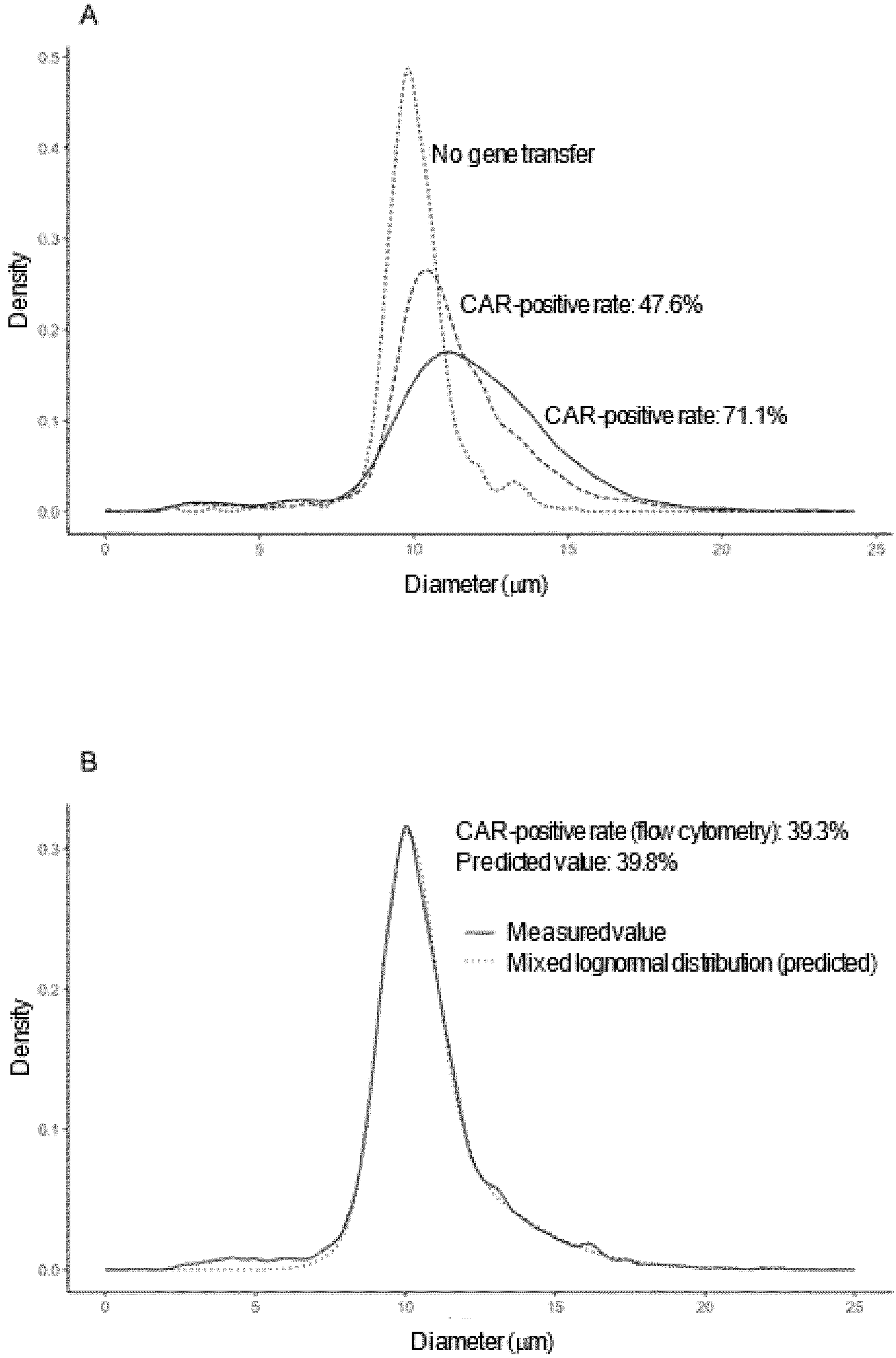
A
Density
0.0
0.1
0.2
0.3
0.4
0.5
0
5
10
15
20
25
Diameter (µm)
No gene transfer
CAR-positive rate: 47.6%
CAR-positive rate: 71.1%
B
Density
0.0
0.1
0.2
0.3
CAR-positive rate (flow cytometry): 39.3%
Predicted value: 39.8%
Measuredvalue
Mixed lognormal distribution (predicted)

Fig. 3

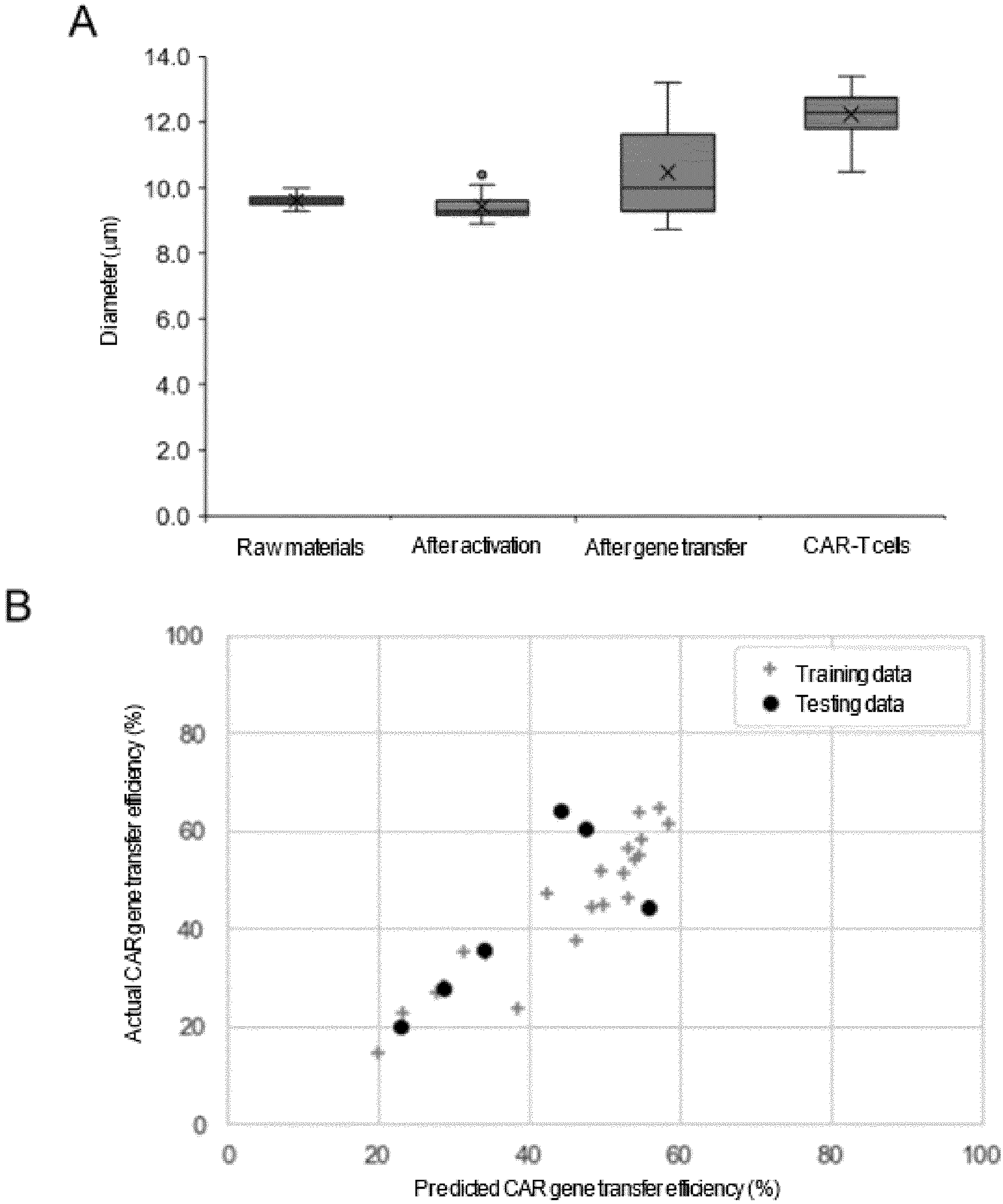
A
14.0
12.0
10.0
8.0
6.0
4.0
2.0
0.0
Diameter (μm)
Raw materials
After activation
After gene transfer
CAR-T cells
B
100
80
60
40
20
0
Actual CAR gene transfer efficiency (%)
Training data
Testing data
0
20
40
60
80
100
Predicted CAR gene transfer efficiency (%)

Fig. 4

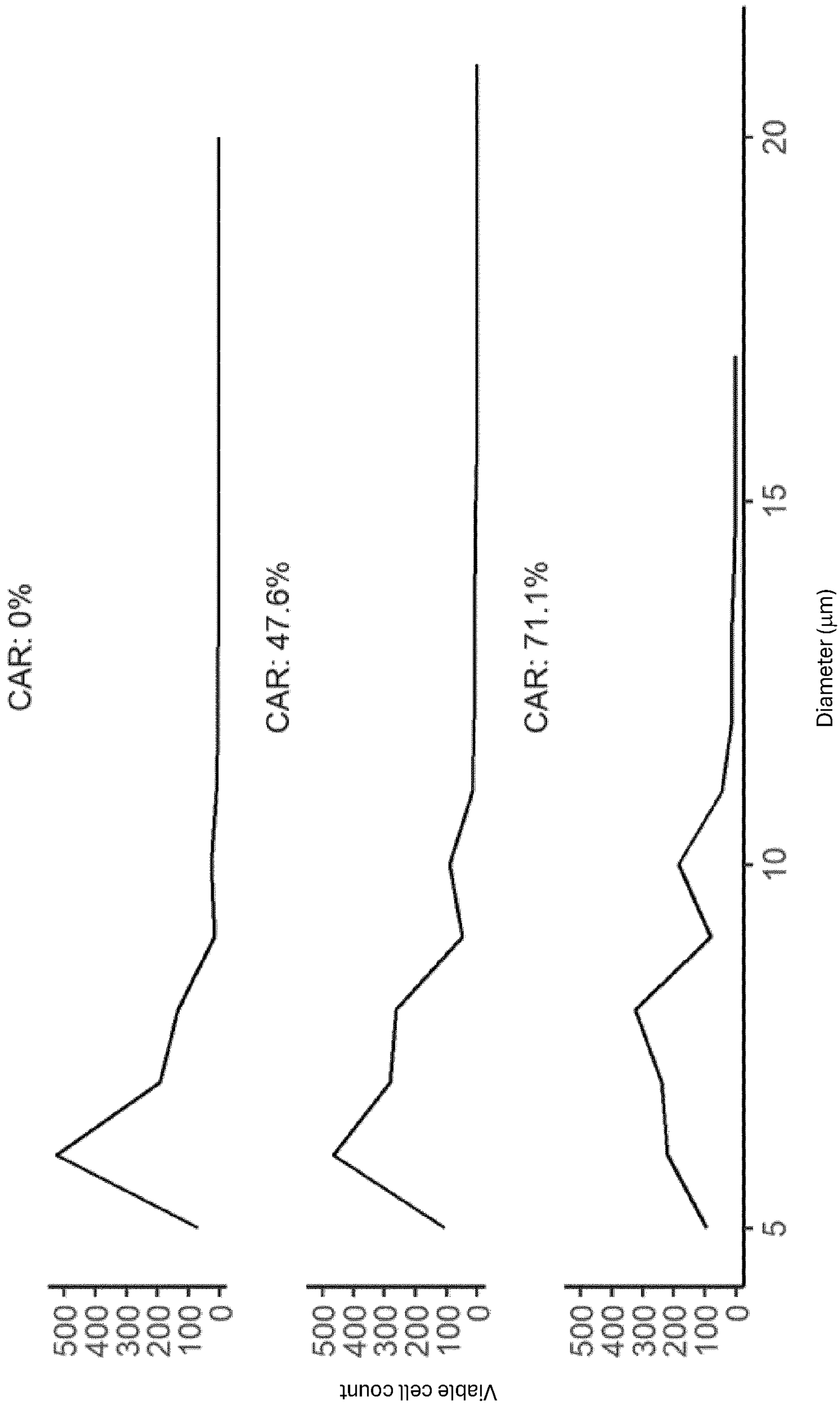
CAR: 0%
CAR: 47.6%
CAR: 71.1%
Viable cell count
500
400
300
200
100
0
Diameter (μm)
5
10
15
20

Fig. 5

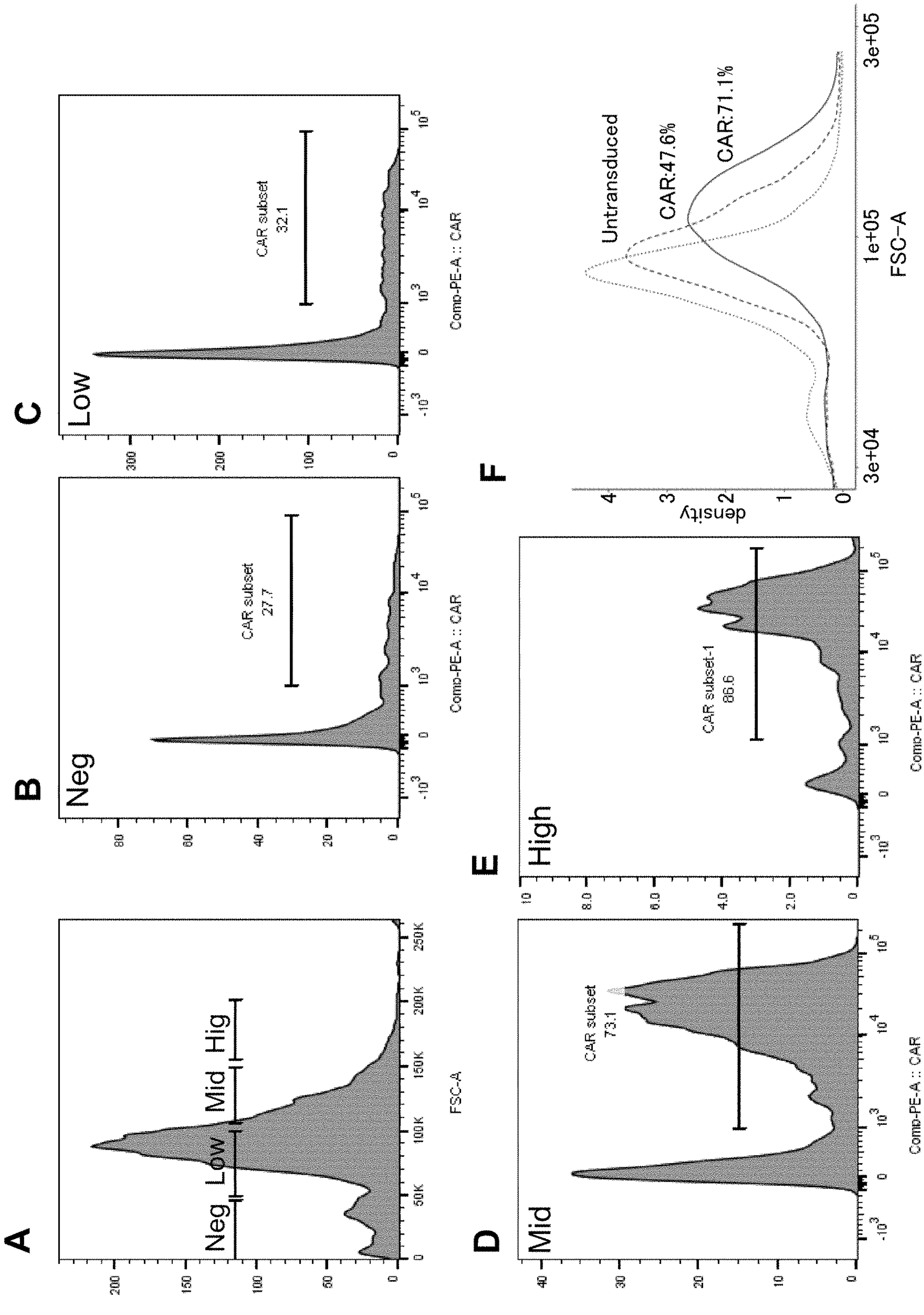
A
Neg
Low
Mid
Hig
FSC-A
B
Neg
CAR subset
27.7
Comp-PE-A :: CAR
C
Low
CAR subset
32.1
Comp-PE-A :: CAR
D
Mid
CAR subset
73.1
Comp-PE-A :: CAR
E
High
CAR subset-1
86.6
Comp-PE-A :: CAR
F
Untransduced
CAR:47.6%
CAR:71.1%
density
FSC-A

Fig. 6

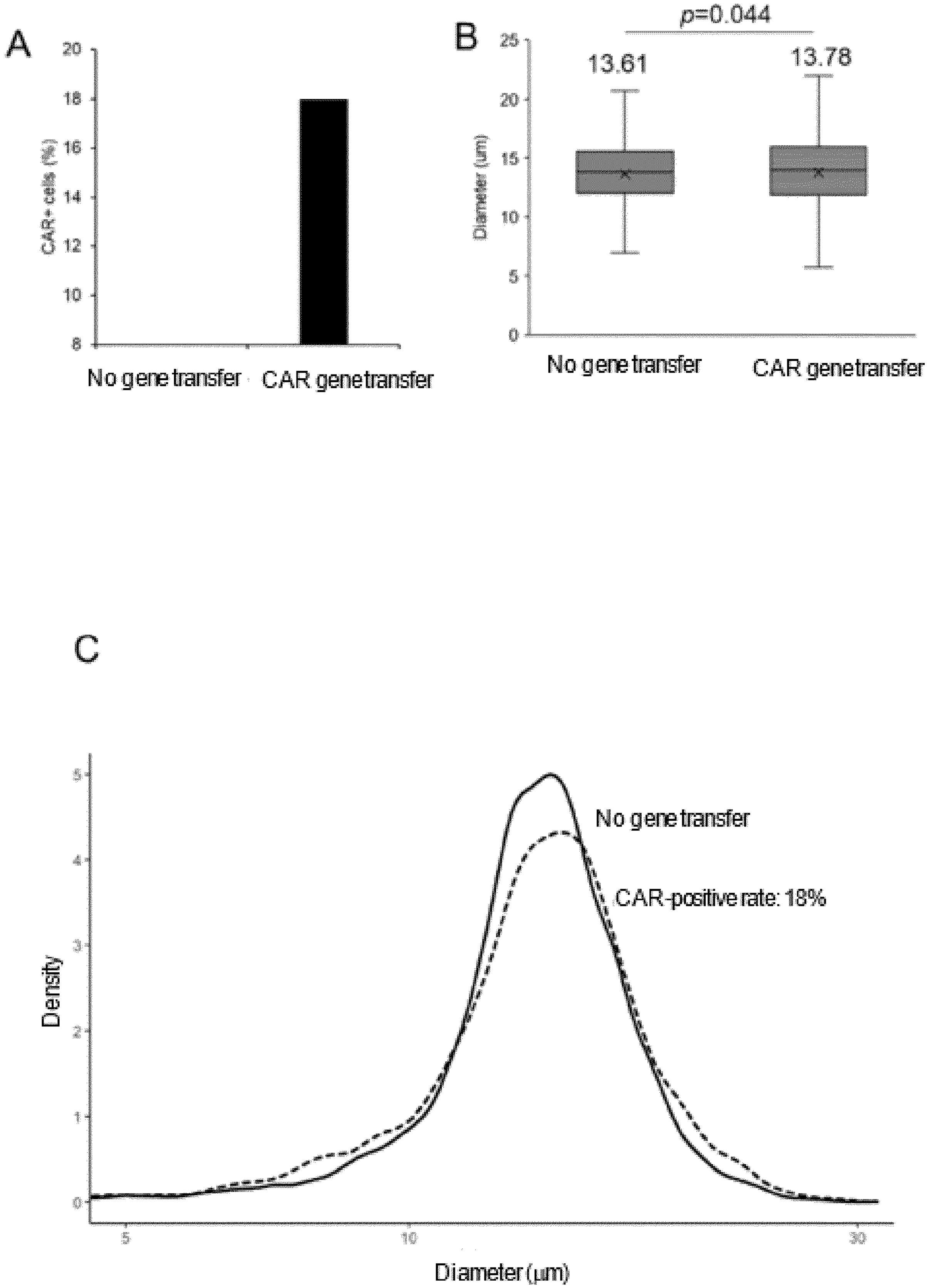
A
20
18
16
14
12
10
8
CAR+ cells (%)
No gene transfer
CAR gene transfer
B
p=0.044
25
20
15
10
5
0
13.61
13.78
Diameter (um)
No gene transfer
CAR gene transfer
C
5
4
3
2
1
0
Density
No gene transfer
CAR-positive rate: 18%
5
10
30
Diameter (μm)

Fig. 7

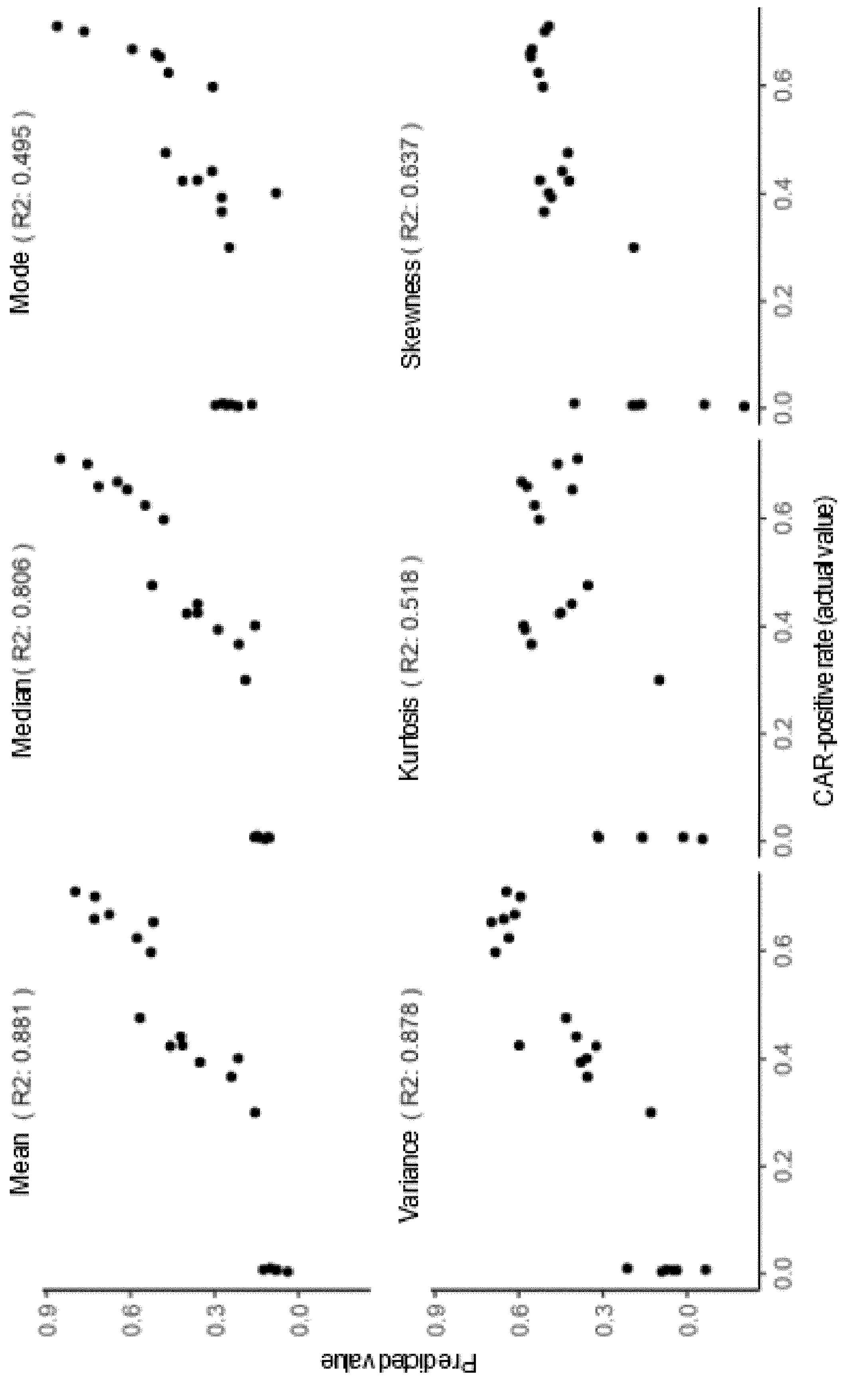
Mean ( R2: 0.881 )
Median ( R2: 0.806 )
Mode ( R2: 0.495 )
Variance ( R2: 0.878 )
Kurtosis ( R2: 0.518 )
Skewness ( R2: 0.637 )
Predicted value
CAR-positive rate (actual value)
0.0
0.2
0.4
0.6
0.3
0.9

Fig. 8

A

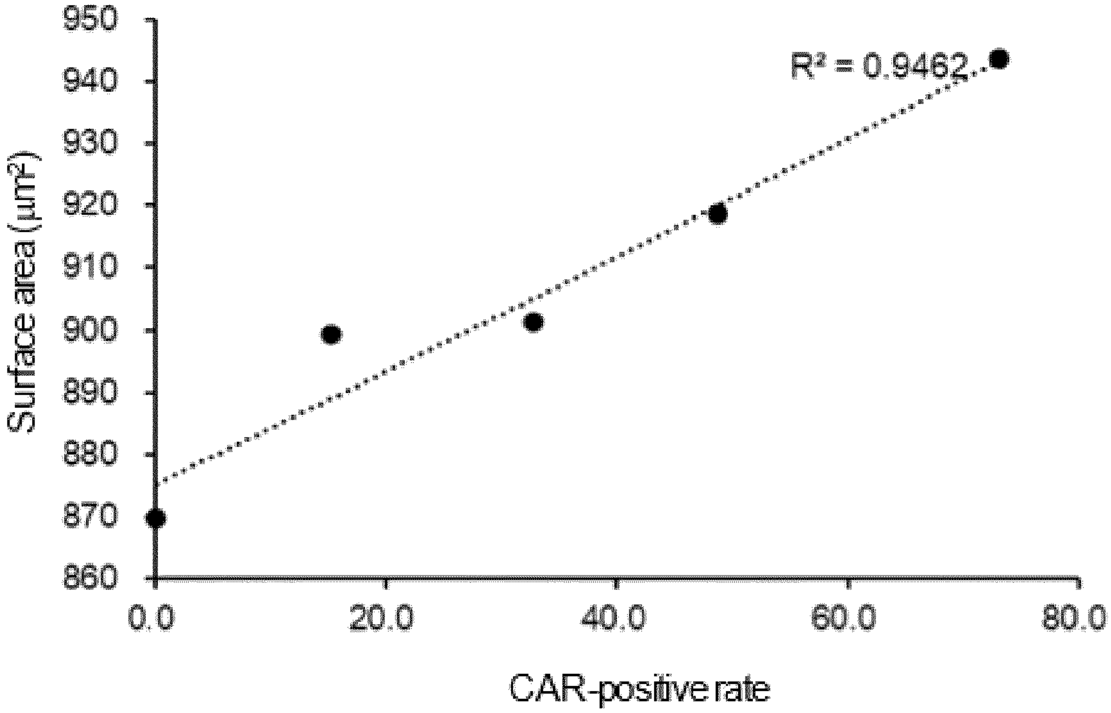
950
940
930
920
910
900
890
880
870
860
Surface area (µm²)
R² = 0.9462
0.0
20.0
40.0
60.0
80.0
CAR-positive rate

B

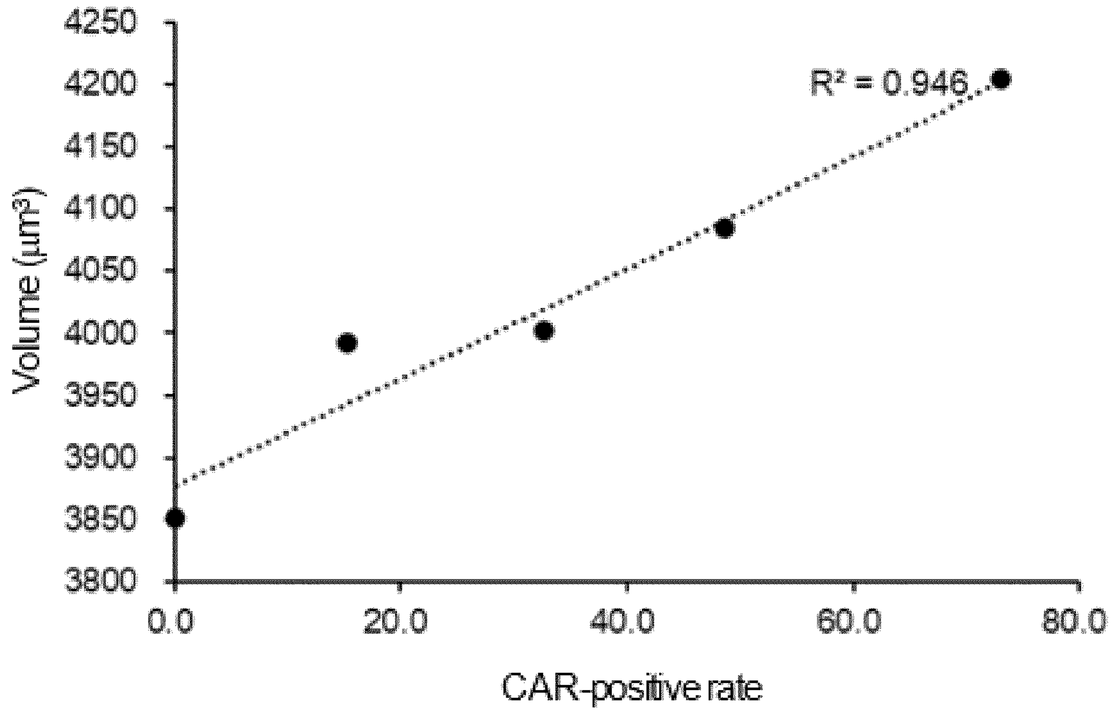
4250
4200
4150
4100
4050
4000
3950
3900
3850
3800
Volume (µm³)
R² = 0.946
0.0
20.0
40.0
60.0
80.0
CAR-positive rate

Fig. 9

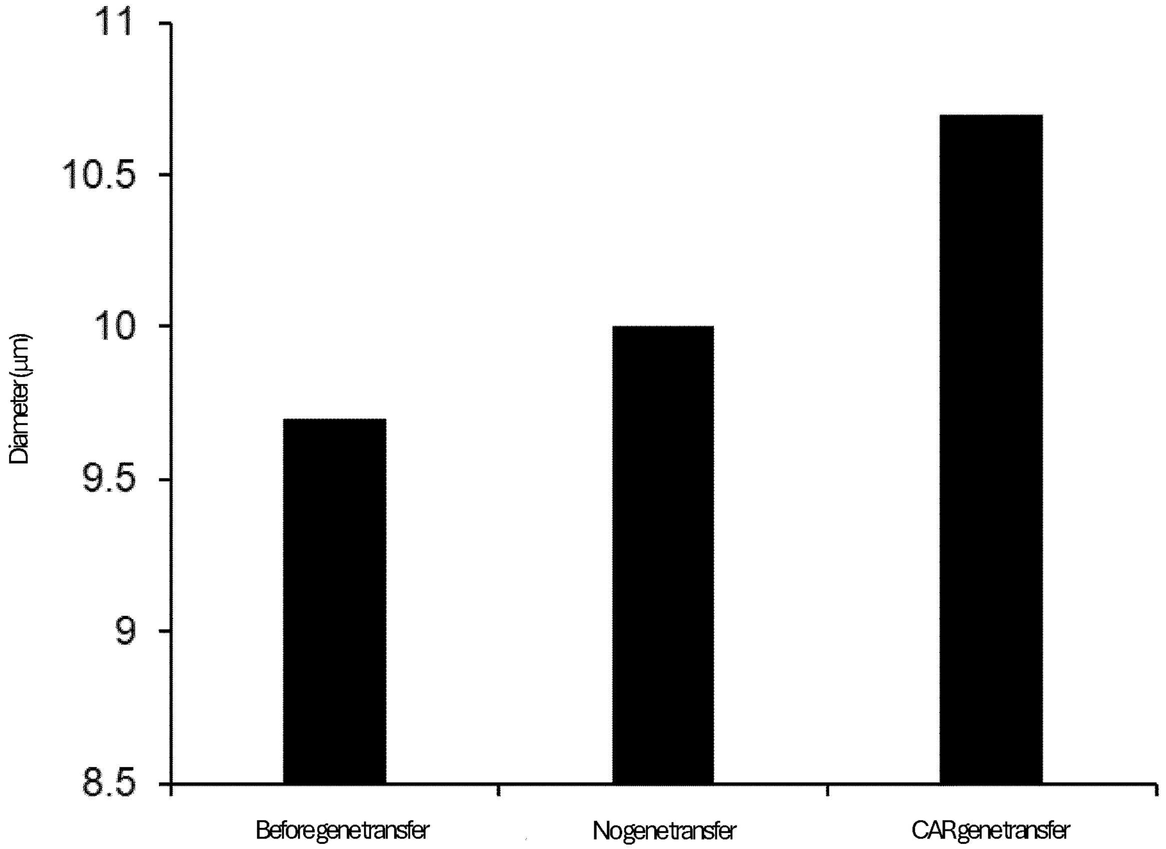
11
10.5
10
9.5
9
8.5
Diameter (μm)
Before gene transfer
No gene transfer
CAR gene transfer

Fig. 10

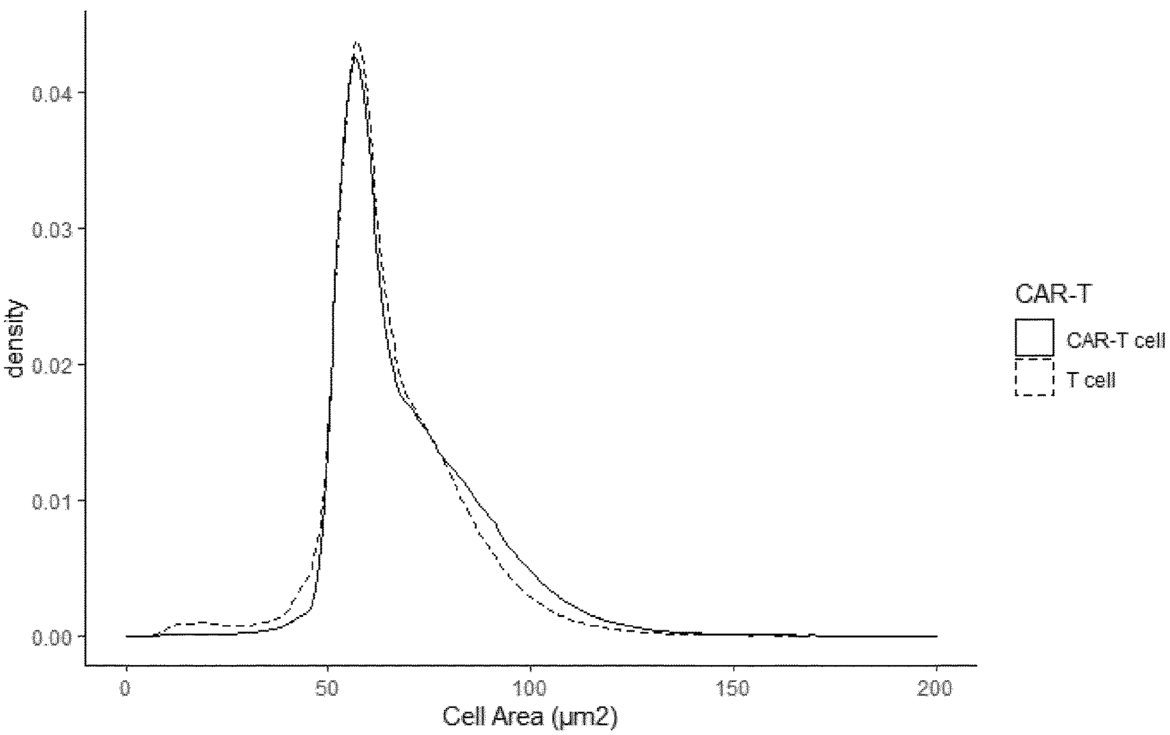
0.04
0.03
0.02
0.01
0.00
density
0
50
100
150
200
Cell Area (μm2)
CAR-T
CAR-T cell
T cell

Fig. 11

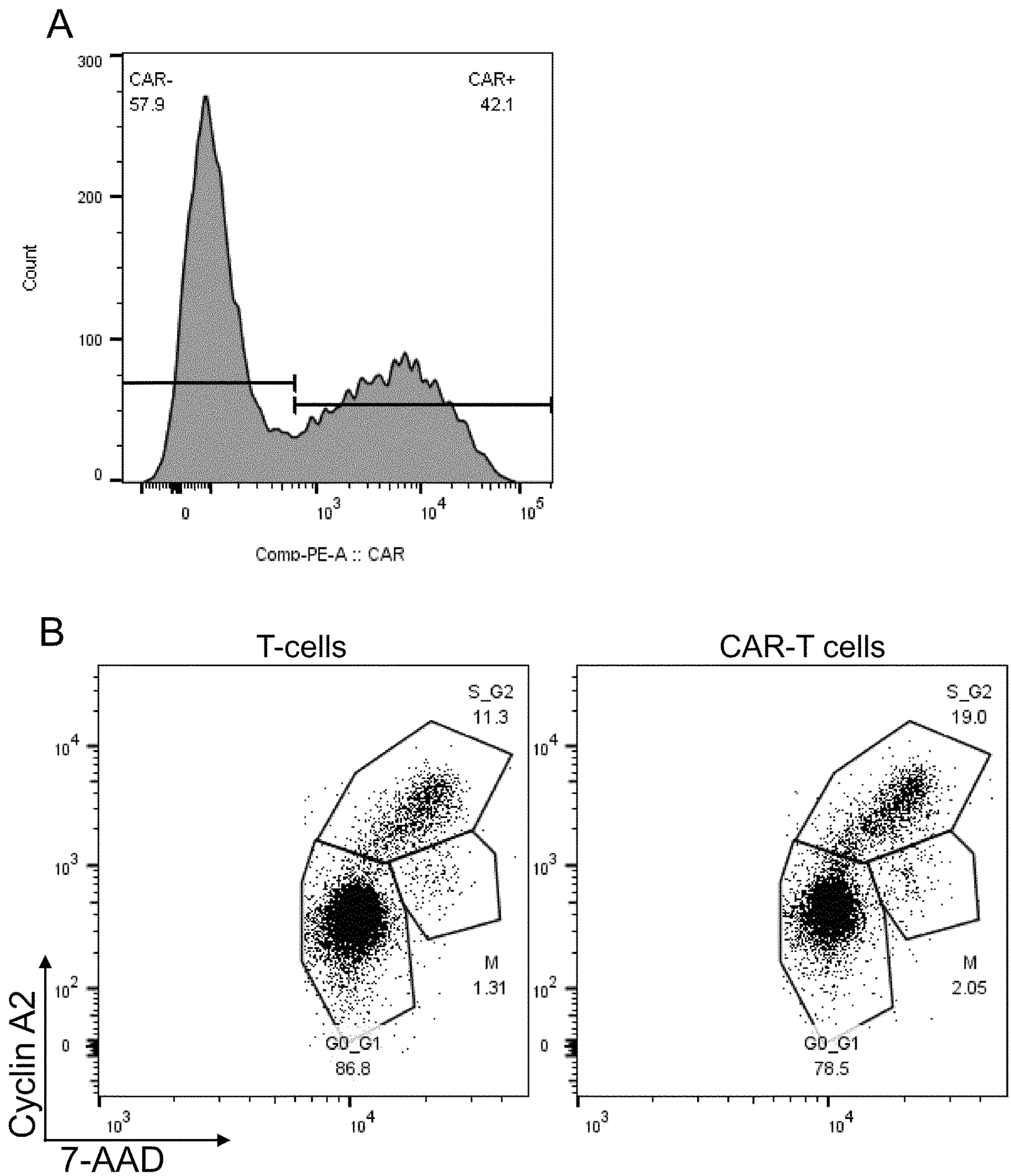
A
300
200
100
0
Count
CAR-
57.9
CAR+
42.1
0
$10^3$
$10^4$
$10^5$
Comp-PE-A :: CAR
B
T-cells
CAR-T cells
S_G2
11.3
19.0
M
1.31
2.05
G0_G1
86.8
78.5
$10^4$
$10^3$
$10^2$
0
Cyclin A2
7-AAD

Fig. 12

A

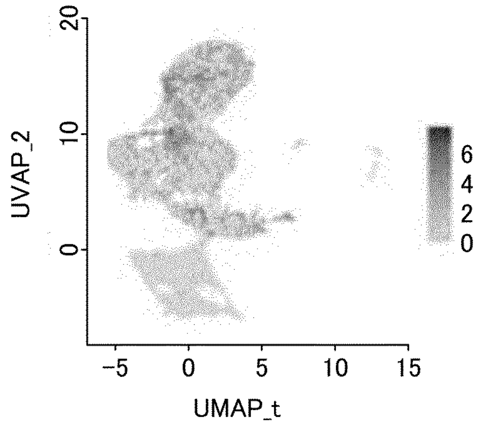


B

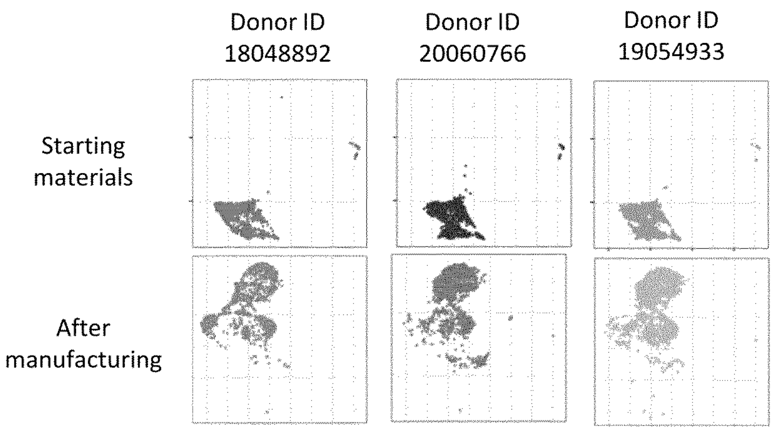


Fig. 13A

A

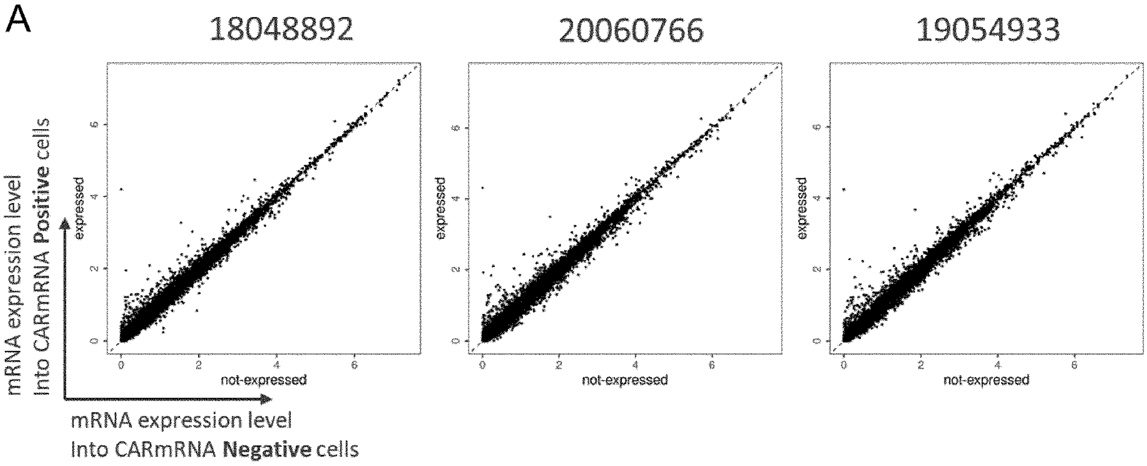

Fig. 13B

B

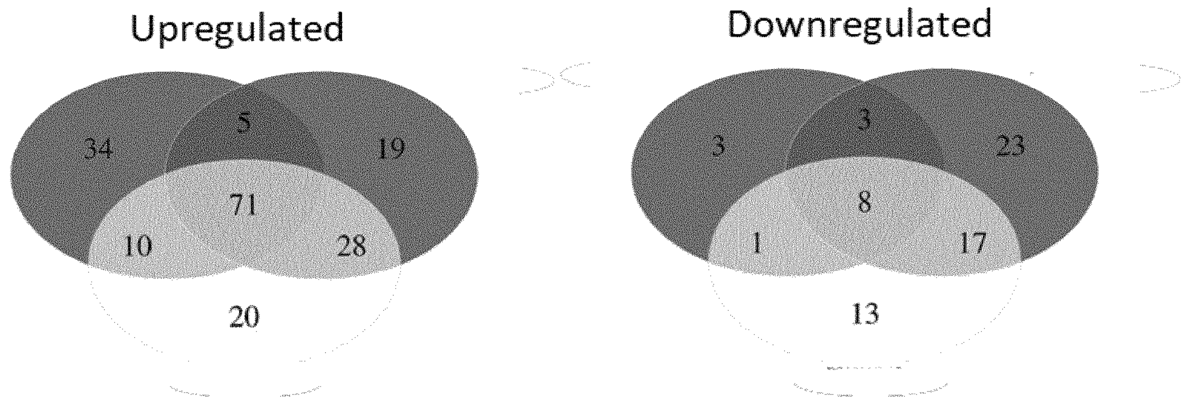


Fig. 13C

C

| Direction | # of common DEGs | Genes |
|---|---|---|
| Up-regulated | 71 | CAR-T, EGFR, HLA-DQA1, HLA-DQA2, CHRNA6, AL606807.1, TOX2, C20orf204, LGALS9, NLGN4Y, HLA-DPA1, TSPAN33, CD74, ITPR1, COL6A1, HLA-DRB5, HLA-DRB1, SEC11C, ST8SIA1, VOPP1, HLA-DRA, BCAT1, BIRC3, TNFRSF4, TP63, KRT7, HLA-DPB1, COL6A2, CD82, DUSP4, ARHGAP10, HMSD, ELL2, FSCN1, CXCL10, TIFA, DBNDD2, CYS1, SDC4, MYB, FAH, HLA-DQB1, STAG3, PTGIR, NFE2L3, NFKB2, IGHM, ARID5A, EBI3, HLA-DOA, CIITA, SLAMF7, FILIP1L, MSC, ADGRE1, DUSP10, RASSF4, SPINT2, HLA-DMA, BTN2A2, HLA-DMB, IER3, GDF11, TPRG1, TRAF1, NFKBIA, TTN, IL22, XCL1, CSF2, CCL1 |
| Down-regulated | 8 | PCED1B-AS1, IFITM1, LINC00861, PTGER2, CD8A, NKG7, CD8B, GZMA |

Fig. 13D

D

GO enrichment analysis

**18048892**

| GO term | p-value |
|---|---|
| immune response | 5.81E-16 |
| regulation of immune system process | 4.83E-15 |
| adaptive immune response | 8.63E-15 |
| positive regulation of immune system process | 1.97E-13 |
| T cell activation | 5.24E-13 |
| immune system process | 7.63E-13 |
| regulation of immune response | 1.48E-12 |
| response to cytokine | 1.99E-12 |
| cellular response to cytokine stimulus | 8.62E-12 |
| cytokine-mediated signaling pathway | 2.09E-11 |

**20060766**

| GO term | p-value |
|---|---|
| immune response | 5.81E-16 |
| regulation of immune system process | 4.83E-15 |
| adaptive immune response | 8.63E-15 |
| positive regulation of immune system process | 1.97E-13 |
| T cell activation | 5.24E-13 |
| immune system process | 7.63E-13 |
| regulation of immune response | 1.48E-12 |
| response to cytokine | 1.99E-12 |
| cellular response to cytokine stimulus | 8.62E-12 |
| cytokine-mediated signaling pathway | 2.09E-11 |

**19054933**

| GO term | p-value |
|---|---|
| immune response | 1.60E-21 |
| cytokine-mediated signaling pathway | 1.17E-19 |
| cellular response to cytokine stimulus | 9.24E-19 |
| response to cytokine | 3.17E-18 |
| immune system process | 4.24E-18 |
| defense response | 4.30E-17 |
| T cell activation | 4.28E-16 |
| adaptive immune response | 1.64E-15 |
| regulation of immune system process | 2.67E-15 |
| lymphocyte activation | 2.74E-15 |

Reactome analysis

**18048892**

| Reactome | p-value |
|---|---|
| Translocation of ZAP-70 to Immunological synapse | 1.03E-11 |
| Phosphorylation of CD3 and TCR zeta chains | 4.97E-11 |
| PD-1 signaling | 7.83E-11 |
| Generation of second messenger molecules | 2.47E-09 |
| Cytokine Signaling in Immune system | 2.07E-08 |
| Interferon gamma signaling | 5.84E-08 |
| MHC class II antigen presentation | 7.70E-08 |
| Immune System | 8.61E-07 |
| Costimulation by the CD28 family | 1.17E-06 |
| Downstream TCR signaling | 1.31E-06 |

**20060766**

| Reactome | p-value |
|---|---|
| Cytokine Signaling in Immune system | 7.56E-14 |
| Translocation of ZAP-70 to Immunological synapse | 1.24E-11 |
| Immune System | 4.94E-11 |
| Phosphorylation of CD3 and TCR zeta chains | 5.95E-11 |
| PD-1 signaling | 9.38E-11 |
| Interferon gamma signaling | 1.13E-09 |
| Generation of second messenger molecules | 2.95E-09 |
| Interleukin-10 signaling | 2.40E-08 |
| Chemokine receptors bind chemokines | 5.03E-08 |
| MHC class II antigen presentation | 9.73E-08 |

**19054933**

| Reactome | p-value |
|---|---|
| Cytokine Signaling in Immune system | 3.11E-13 |
| Immune System | 6.88E-12 |
| Translocation of ZAP-70 to Immunological synapse | 1.92E-11 |
| Phosphorylation of CD3 and TCR zeta chains | 9.18E-11 |
| Interferon gamma signaling | 1.05E-10 |
| PD-1 signaling | 1.45E-10 |
| Interferon Signaling | 5.03E-10 |
| Generation of second messenger molecules | 4.52E-09 |
| MHC class II antigen presentation | 1.70E-07 |
| Downstream TCR signaling | 2.01E-07 |

Fig. 14A

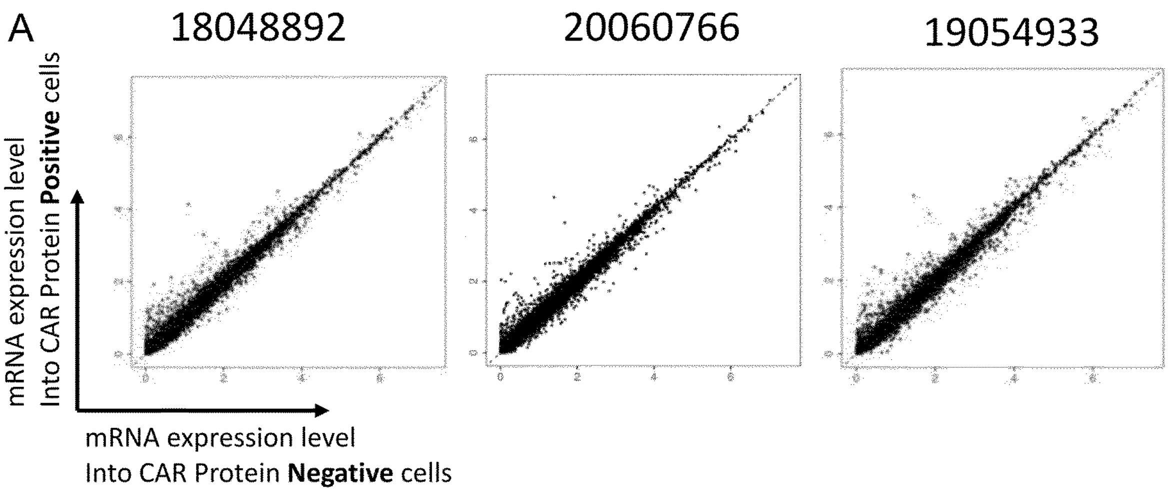


Fig. 14B

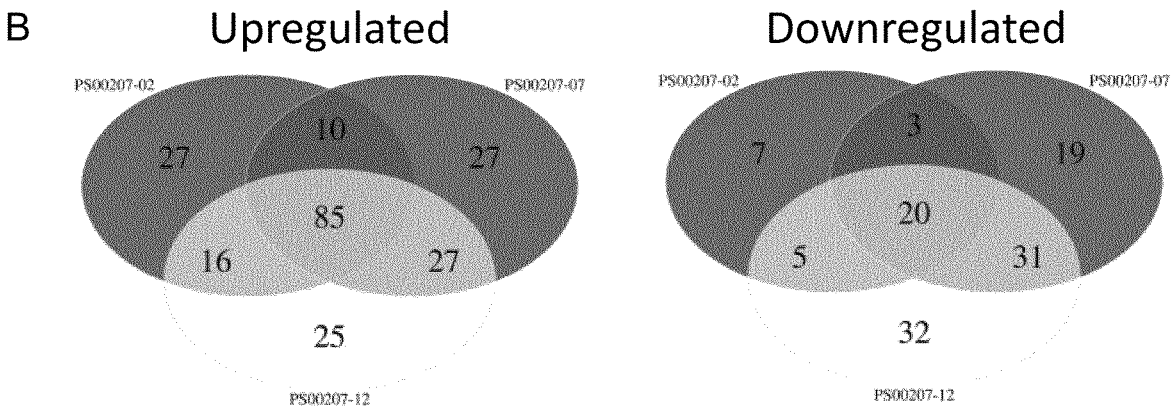

Fig. 14C

C

| Direction | # of common DEGs | Genes |
|---|---|---|
| Up-regulated | 85 | CAR-T, EGFR, HLA-DQA1, CHRNA6, HLA-DQA2, AL606807.1, TOX2, LGALS9, C20orf204, HLA-DPA1, CD74, TSPAN33, NLGN4Y, HLA-DRA, VOPP1, COL6A1, ASB2, SEC11C, TP63, HLA-DRB1, COL6A2, HLA-DRB5, PKM, TNFRSF4, FSCN1, CD82, BIRC3, KRT7, ITPR1, ST8SIA1, DUSP4, GPX4, SDC4, BCAT1, HLA-DPB1, HMSD, ARHGAP10, EBI3, ELL2, CPM, DBNDD2, STAG3, CXCL10, TIFA, PTGIR, ARID5A, HLA-DQB1, NFKB2, HLA-DOA, FAH, CYS1, MYB, TMEM200A, IGHM, RGS1, CIITA, ADGRE1, NFE2L3, ICAM1, DUSP10, MSC, IER3, ZG16B, FILIP1L, ANKRD33B, SPINT2, HLA-DMA, IL21, PTPRK, TCF7, GDF11, TRAF1, TPRG1, SLAMF7, BTN2A2, RASSF4, LIF, RDH10, ATF5, NFKBIA, MIR155HG, XCL1, CSF2, IL22, CCL1 |
| Down-regulated | 20 | PCED1B-AS1, IFITM1, S100A10, PTGER2, HCST, CD44, LINC00861, SAMD3, IL7R, NKG7, CD8A, CD8B, RNF157, GZMA, TIMP1, FCMR, KLRK1, ZNF683, PRF1, RIN3 |

Fig. 14D

D

**GO enrichment analysis**

| 18048892 GO term | p-value | 20060766 GO term | p-value | 19054933 GO term | p-value |
|---|---|---|---|---|---|
| immune response | 2.79E-23 | immune response | 3.70E-25 | immune system process | 2.22E-26 |
| immune system process | 1.58E-19 | regulation of immune system process | 1.31E-22 | immune response | 4.25E-26 |
| regulation of immune system process | 3.28E-17 | immune system process | 2.45E-22 | regulation of immune system process | 1.04E-24 |
| T cell activation | 1.75E-16 | T cell activation | 2.76E-21 | cellular response to cytokine stimulus | 1.15E-24 |
| adaptive immune response | 1.94E-16 | cellular response to cytokine stimulus | 9.30E-21 | cytokine-mediated signaling pathway | 1.75E-24 |
| cell activation | 5.29E-16 | lymphocyte activation | 1.26E-20 | T cell activation | 4.05E-24 |
| lymphocyte activation | 7.04E-16 | cytokine-mediated signaling pathway | 1.27E-20 | response to cytokine | 1.19E-23 |
| regulation of immune response | 1.26E-15 | response to cytokine | 5.46E-20 | lymphocyte activation | 1.56E-23 |
| cytokine-mediated signaling pathway | 7.35E-15 | regulation of immune response | 4.63E-18 | regulation of T cell activation | 3.57E-22 |
| response to stimulus | 1.64E-14 | regulation of cell-cell adhesion | 1.33E-17 | leukocyte cell-cell adhesion | 1.65E-21 |

**Reactome analysis**

| 18048892 Reactome | p-value | 20060766 Reactome | p-value | 19054933 Reactome | p-value |
|---|---|---|---|---|---|
| Immune System | 7.29E-12 | Cytokine Signaling in Immune system | 6.04E-14 | Cytokine Signaling in Immune system | 9.23E-18 |
| Translocation of ZAP-70 to Immunological synapse | 2.27E-11 | Immune System | 1.51E-12 | Immune System | 9.81E-18 |
| Phosphorylation of CD3 and TCR zeta chains | 1.08E-10 | Translocation of ZAP-70 to Immunological synapse | 5.36E-11 | Immunoregulatory interactions between a Lymphoid and a non-Lymphoid cell | 1.10E-11 |
| PD-1 signaling | 1.71E-10 | Phosphorylation of CD3 and TCR zeta chains | 2.55E-10 | Translocation of ZAP-70 to Immunological synapse | 6.66E-11 |
| Cytokine Signaling in Immune system | 2.04E-10 | PD-1 signaling | 4.01E-10 | Interferon gamma signaling | 2.50E-10 |
| Interferon gamma signaling | 2.88E-09 | Immunoregulatory interactions between a Lymphoid and a non-Lymphoid cell | 4.59E-10 | Phosphorylation of CD3 and TCR zeta chains | 3.17E-10 |
| Generation of second messenger molecules | 5.33E-09 | Interferon gamma signaling | 7.39E-10 | PD-1 signaling | 4.98E-10 |
| Interferon Signaling | 1.74E-07 | Interleukin-10 signaling | 1.07E-09 | Signaling by Interleukins | 9.17E-09 |
| MHC class II antigen presentation | 2.11E-07 | Signaling by Interleukins | 1.06E-08 | Interferon Signaling | 9.70E-09 |
| Costimulation by the CD28 family | 2.43E-06 | Generation of second messenger molecules | 1.23E-08 | Generation of second messenger molecules | 1.53E-08 |

Fig. 14E

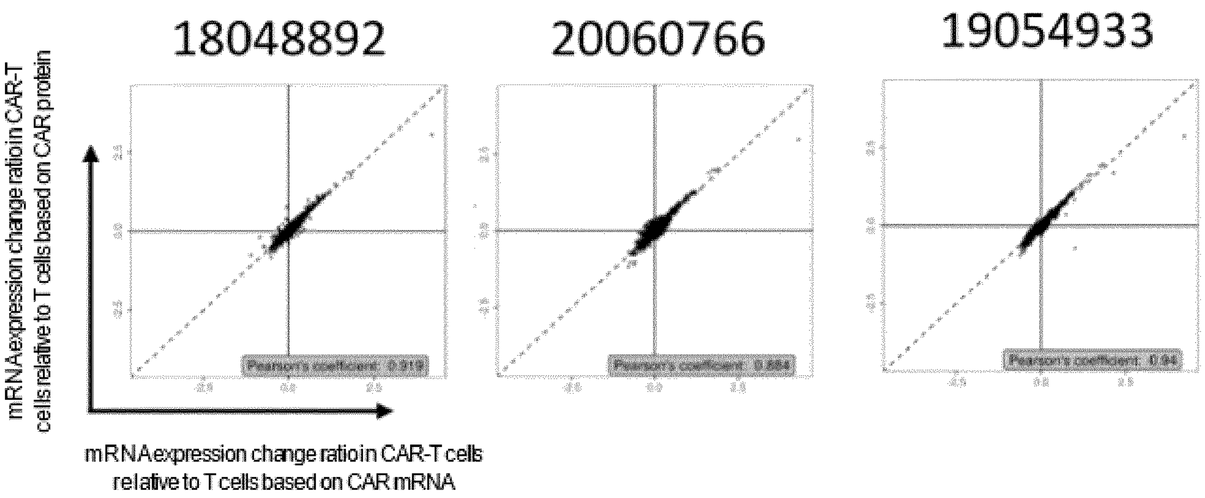


Fig. 15A

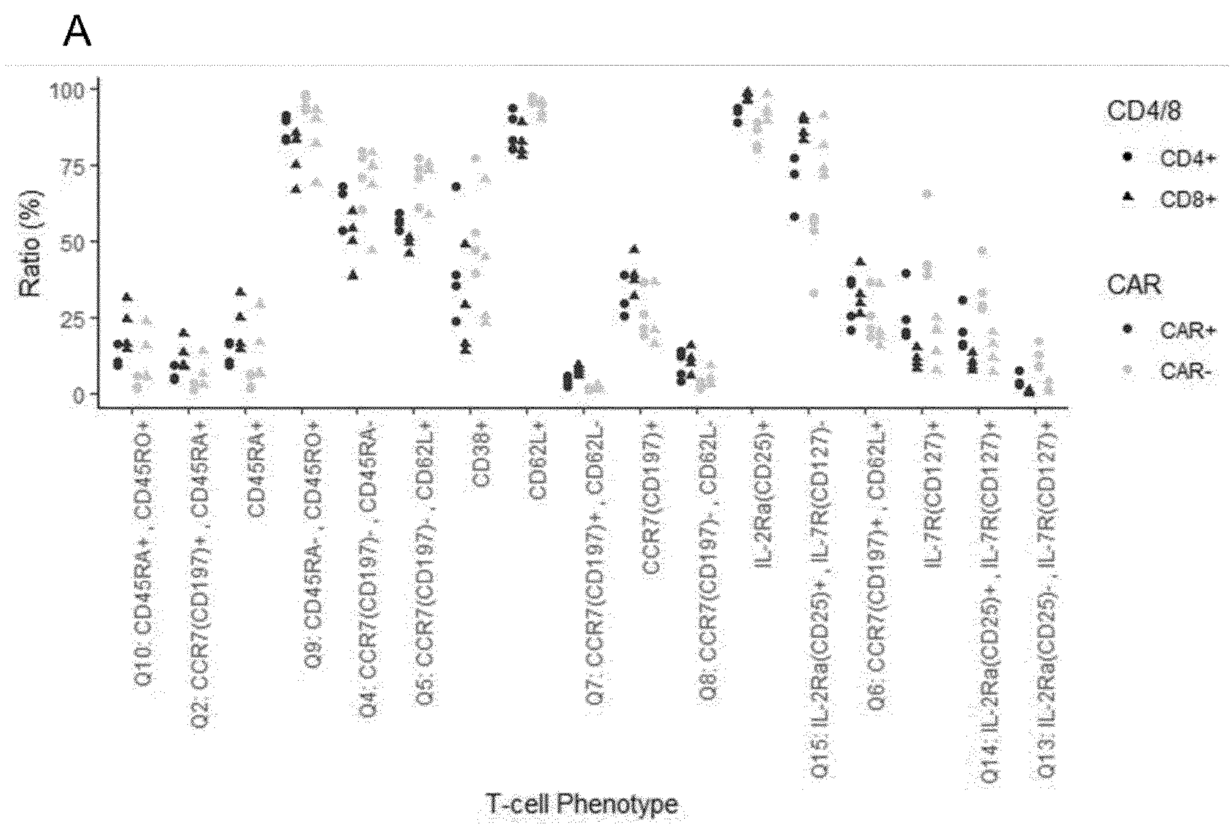

Fig. 15B

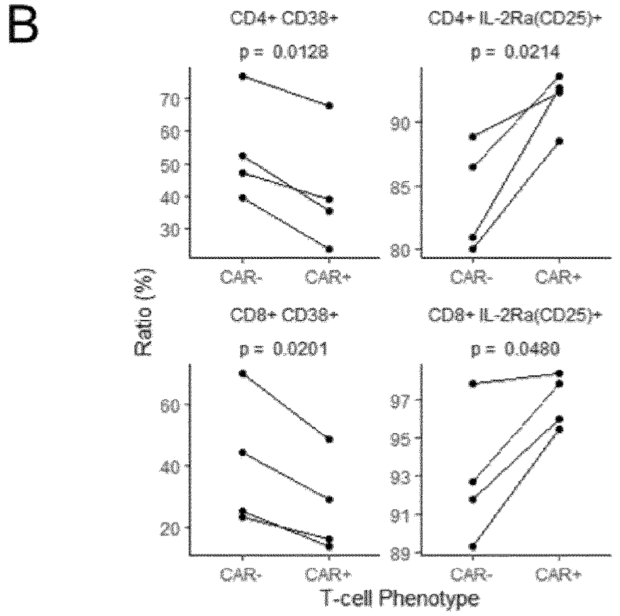
B
CD4+ CD38+
p = 0.0128
CD4+ IL-2Ra(CD25)+
p = 0.0214
CD8+ CD38+
p = 0.0201
CD8+ IL-2Ra(CD25)+
p = 0.0480
Ratio (%)
CAR-
CAR+
T-cell Phenotype

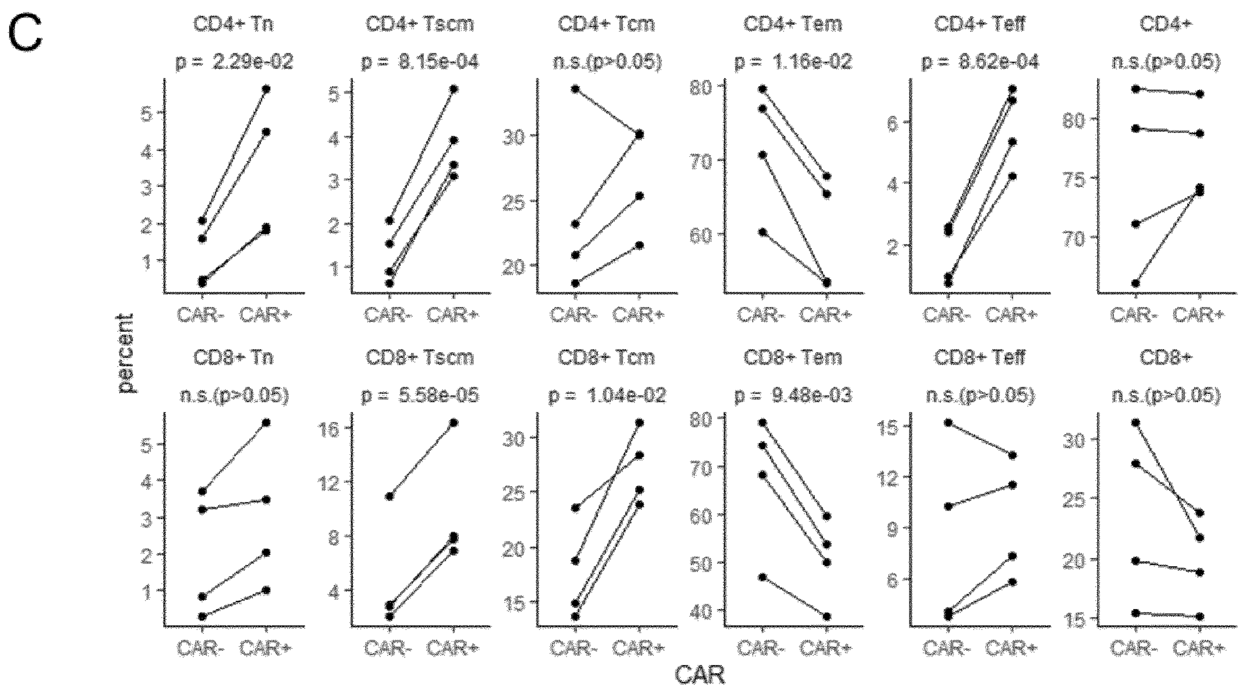
C
CD4+ Tn
p = 2.29e-02
CD4+ Tscm
p = 8.15e-04
CD4+ Tcm
n.s.(p>0.05)
CD4+ Tem
p = 1.16e-02
CD4+ Teff
p = 8.62e-04
CD4+
n.s.(p>0.05)
CD8+ Tn
n.s.(p>0.05)
CD8+ Tscm
p = 5.58e-05
CD8+ Tcm
p = 1.04e-02
CD8+ Tem
p = 9.48e-03
CD8+ Teff
n.s.(p>0.05)
CD8+
n.s.(p>0.05)
percent
CAR-
CAR+
CAR

Fig. 16

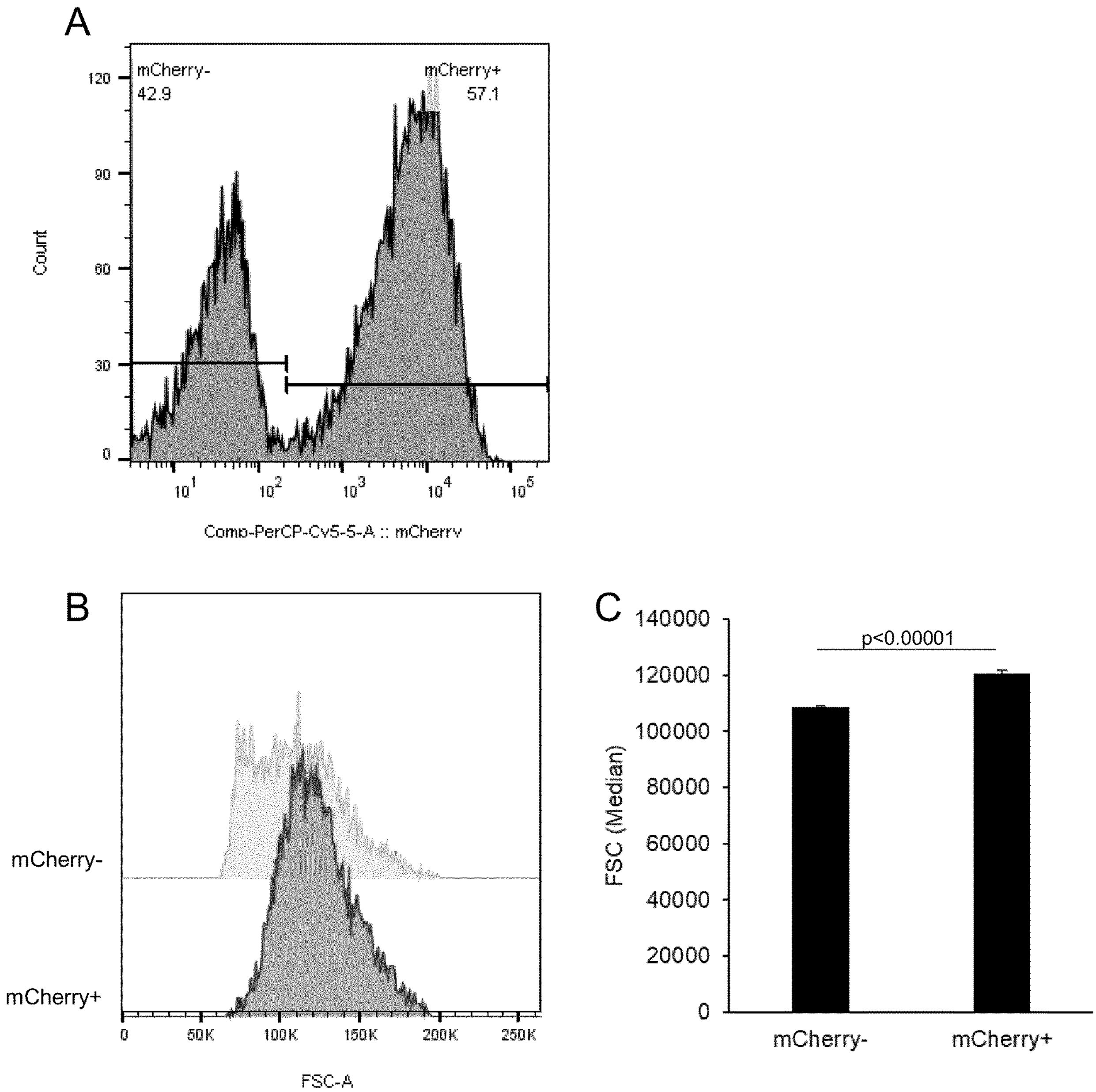
A
mCherry-
42.9
mCherry+
57.1
Count
120
90
60
30
0
$10^1$
$10^2$
$10^3$
$10^4$
$10^5$
Comp-PerCP-Cy5-5-A :: mCherry
B
mCherry-
mCherry+
0
50K
100K
150K
200K
250K
FSC-A
C
p<0.00001
FSC (Median)
140000
120000
100000
80000
60000
40000
20000
0
mCherry-
mCherry+

Fig. 17

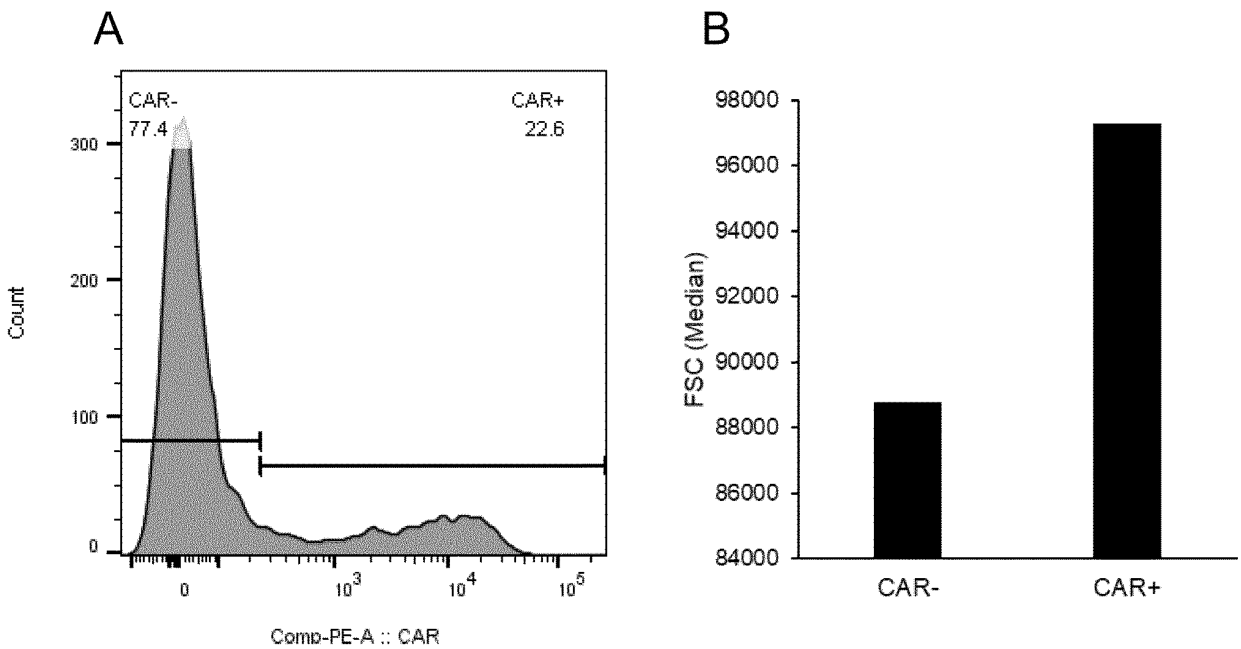
A
CAR-
77.4
CAR+
22.6
Count
300
200
100
0
0
$10^3$
$10^4$
$10^5$
Comp-PE-A :: CAR
B
98000
96000
94000
92000
90000
88000
86000
84000
FSC (Median)
CAR-
CAR+

Fig. 18

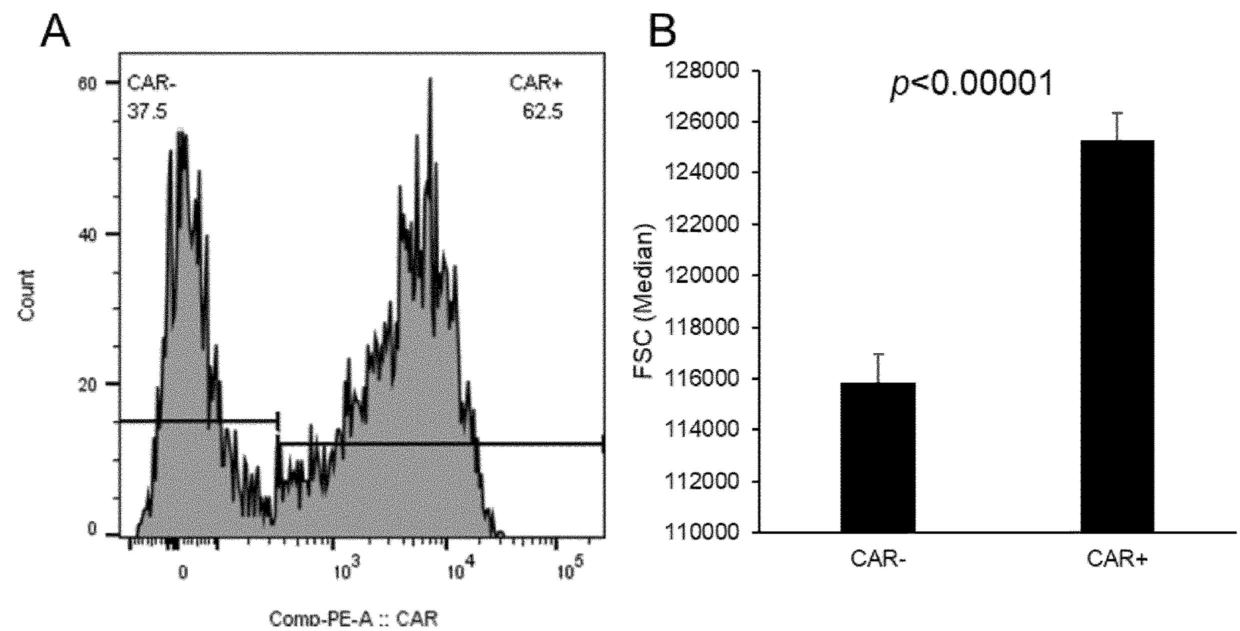
A
CAR-
37.5
CAR+
62.5
Count
60
40
20
0
0
$10^3$
$10^4$
$10^5$
Comp-PE-A :: CAR
B
p<0.00001
128000
126000
124000
122000
120000
118000
116000
114000
112000
110000
FSC (Median)
CAR-
CAR+

Fig. 19

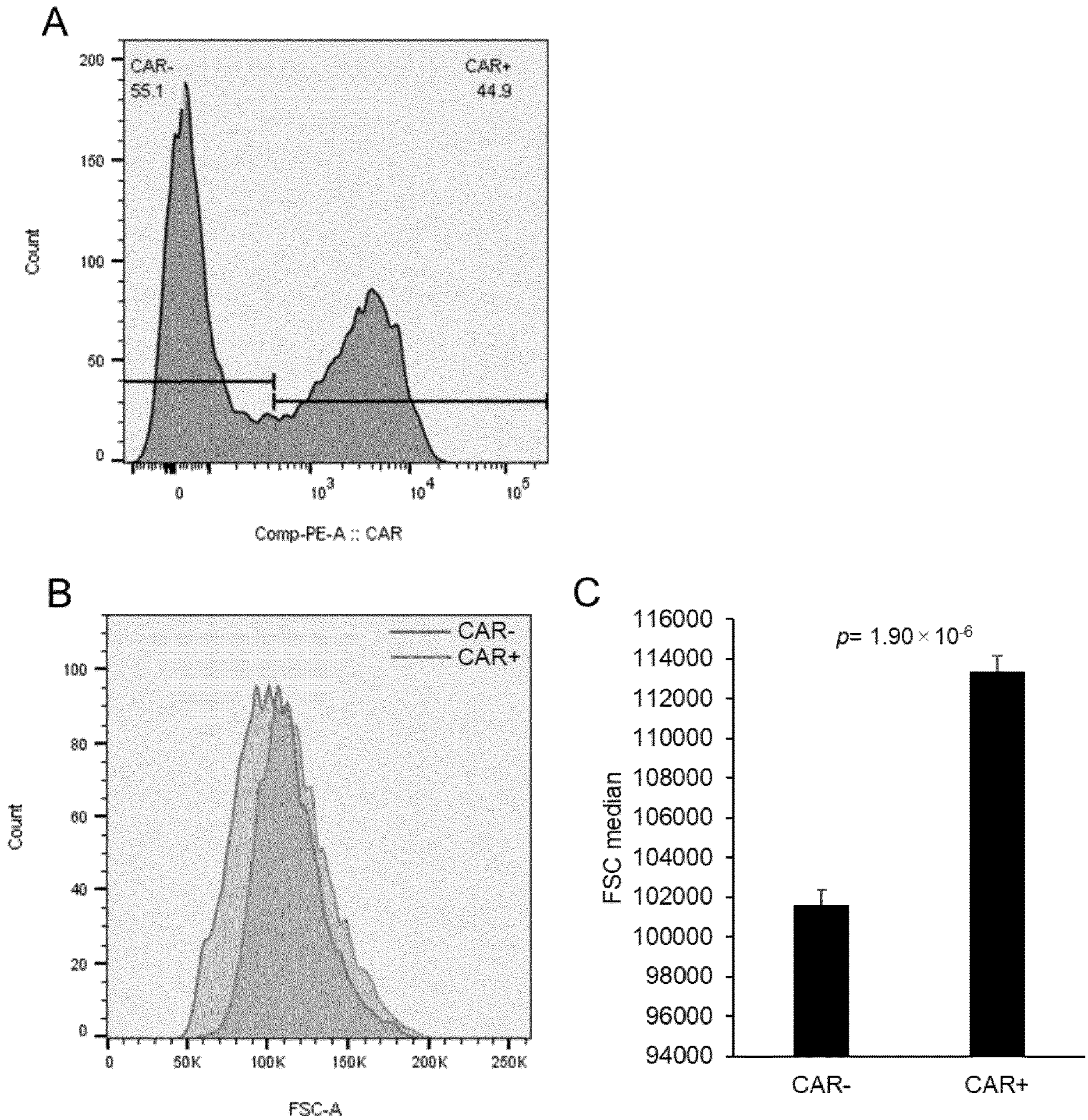
A
CAR-
55.1
CAR+
44.9
Count
200
150
100
50
0
0
$10^3$
$10^4$
$10^5$
Comp-PE-A :: CAR
B
CAR-
CAR+
Count
100
80
60
40
20
0
0
50K
100K
150K
200K
250K
FSC-A
C
$p$= 1.90 × $10^{-6}$
FSC median
116000
114000
112000
110000
108000
106000
104000
102000
100000
98000
96000
94000
CAR-
CAR+

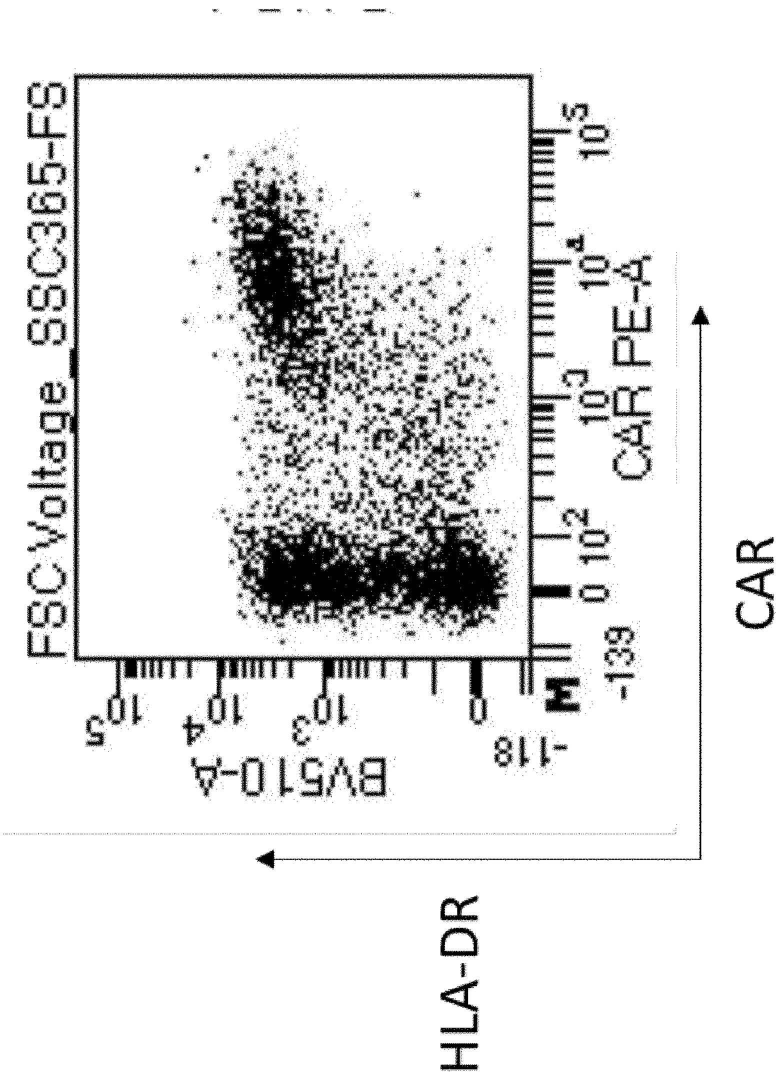


Fig. 20

**INTERNATIONAL SEARCH REPORT**

International application No.
**PCT/JP2022/047423**

**A. CLASSIFICATION OF SUBJECT MATTER**

***C12Q 1/04***(2006.01)i; ***C12N 5/0783***(2010.01)i; ***C12N 5/10***(2006.01)i; ***C12N 15/09***(2006.01)i; ***G01N 33/50***(2006.01)i
FI: C12Q1/04 ZNA; G01N33/50 Z; C12N15/09 Z; C12N5/10; C12N5/0783

According to International Patent Classification (IPC) or to both national classification and IPC

**B. FIELDS SEARCHED**

Minimum documentation searched (classification system followed by classification symbols)

C12Q1/04; C12N5/0783; C12N5/10; C12N15/09; G01N33/50

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | WANG, Yongtao et al. Regulation of gene transfection by cell size, shape and elongation on micropatterned surfaces. Journal of Materials Chemistry B. May 2021, vol. 9, pp. 4329-4339<br>abstract, fig. 2 | 1-17 |
| Y | JP 2020-525537 A (EMORY UNIVERSITY) 27 August 2020 (2020-08-27)<br>paragraphs [0145], [0146] | 1-12, 14-17 |
| A | paragraphs [0145], [0146] | 13 |
| Y | JP 2020-528284 A (LONZA WALKERSVILLE INC) 24 September 2020 (2020-09-24)<br>paragraphs [0134], [0351] | 1-12, 14-17 |
| A | paragraphs [0134], [0351] | 13 |
| Y | WO 2020/102676 A1 (CELGENE CORPORATION) 22 May 2020 (2020-05-22)<br>paragraph [0070] | 1-12, 14-17 |
| A | paragraph [0070] | 13 |
| Y | JP 2006-345852 A (VIRXSYS CORP) 28 December 2006 (2006-12-28)<br>paragraph [0070] | 1-12, 14-17 |
| A | paragraph [0070] | 13 |

☐ Further documents are listed in the continuation of Box C.　☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 March 2023** | **14 March 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/047423**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

“The form of Annex C/ST.25 text file” above shall read as “the form of ST.26.”.

Form PCT/ISA/210 (continuation of first sheet) (January 2015)

**INTERNATIONAL SEARCH REPORT**
**Information on patent family members**

International application No.
**PCT/JP2022/047423**

| Patent document cited in search report | Publication date (day/month/year) | Patent family member(s) | Publication date (day/month/year) |
|---|---|---|---|
| JP 2020-525537 A | 27 August 2020 | US 2020/0179450 A1<br>paragraphs [0168], [0169]<br>WO 2018/231871 A1<br>EP 3638261 A1<br>CN 110944652 A | |
| JP 2020-528284 A | 24 September 2020 | US 2019/0169572 A1<br>paragraphs [0180], [0411]<br>WO 2019/046766 A2<br>EP 3662056 A2<br>KR 10-2020-0054215 A<br>CN 111373030 A | |
| WO 2020/102676 A1 | 22 May 2020 | JP 2022-513045 A<br>paragraph [0070]<br>US 2022/0017862 A1<br>EP 3880802 A1<br>CN 113056558 A1<br>KR 10-2021-0092743 A | |
| JP 2006-345852 A | 28 December 2006 | US 2007/0036783 A1<br>paragraph [0099]<br>WO 2006/138670 A2<br>KR 10-2006-0131596 A<br>CN 1880446 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- JP 2021209987 A
- JP 2022158190 A

### Non-patent literature cited in the description

- *Cytometry Part B: Clinical Cytometry,* vol. 100 (2), 218-224
- **HENNING, AMANDA N et al.** *Nature Reviews Immunology,* 2018, vol. 18 (5), 340-356